# EUROPEAN PATENT APPLICATION

(11) **EP 1 336 619 A2**
(43) Date of publication of application: **20.08.2003**
(21) Application number: 03003651.1
(22) Date of filing: 18.02.2003
(51) Int. Cl.: C07K 14/705, C12N 15/62, A61K 38/17, A61K 48/00, C07K 16/28

(54) **Combination of a HVEM-LIGHT inhibitor and an immunosuppressive agent in the treatment or prevention of immune disorders**

(30) Priority: 19.02.2002 US 358463 P
(71) Applicant: MILLENIUM PHARMACEUTICALS, INC., Cambridge, MA 02139 (US)
(72) Inventor: Fraser, Christopher C., Lexington, MA 02421 (US); Hancock, Wayne, Philadelphia, PA 19147 (US)
(74) Representative: Schüssler, Andrea, Dr.

(57) **Abstract**

The present invention provides methods of suppressing immune disorders such as allograft rejection in a patient by a combination of inhibition of the binding of LIGHT to HVEM and immunosuppressive therapy. Various therapeutic regimens are provided. The present invention further provides kits and pharmaceutical compositions useful in the present methods.

SEQUENCE LISTING

<110> Fraser, Christopher
      Hancock, Wayne

<120> METHODS AND COMPOSITIONS FOR THE TREATMENT OR PREVENTION OF IMMUNE
      DISORDERS USING COMBINATION THERAPY

<130> 7853-255-888

<160> 23

<170> PatentIn version 3.1

<210> 1
<211> 1724
<212> DNA
<213> Homo sapiens

<220>
<221> CDS
<222> (294)..(1145)
<223>

<400> 1
ccttcatacc ggcccttccc ctcggctttg cctggacagc tcctgcctcc cgcagggccc      60
acctgtgtcc cccagcgccg ctccacccag caggcctgag cccctctctg ctgccagaca     120
ccccctgctg cccactctcc tgctgctcgg gttctgaggc acagcttgtc acaccgaggc     180
ggattctctt tctctttctc ttctggccca cagccgcagc aatggcgctg agttcctctg     240
ctggagttca tcctgctagc tgggttcccg agctgccggt ctgagcctga ggc atg        296
gag cct cct gga gac tgg ggg cct cct ccc tgg aga tcc acc ccc aga       344
acc gac gtc ttg agg ctg gtg ctg tat ctc acc ttc ctg gga gcc ccc       392
tgc tac gcc cca gct ctg ccg tcc tgc aag gag gac gag tac cca gtg       440
ggc tcc gag tgc tgc ccc aag tgc agt cca ggt tat cgt gtg aag gag       488
gcc tgc ggg gag ctg acg ggc aca gtg tgt gaa ccc tgc cct cca ggc       536
acc tac att gcc cac ctc aat ggc cta agc aag tgt ctg cag tgc caa       584
atg tgt gac cca gcc atg ggc ctg cgc gcg agc cgg aac tgc tcc agg       632
aca gag aac gcc gtg tgt ggc tgc agc cca ggc cac ttc tgc atc gtc       680
cag gac ggg gac cac tgc gcc gcg tgc cgc gct tac gcc acc tcc agc       728
ccg ggc cag agg gtg cag aag gga ggc acc gag agt cag gac acc ctg       776
tgt cag aac tgc ccc ccg ggg acc ttc tct ccc aat ggg acc ctg gag 824
gaa tgt cag cac cag acc aag tgc agc tgg ctg gtg acg aag gcc gga 872
gct ggg acc agc agc tcc cac tgg gta tgg tgg ttt ctc tca ggg agc 920
ctc gtc atc gtc att gtt tgc tcc aca gtt ggc cta atc ata tgt gtg 968
aaa aga aga aag cca agg ggt gat gta gtc aag gtg atc gtc tcc gtc 1016
cag cgg aaa aga cag gag gca gaa ggt gag gcc aca gtc att gag gcc 1064
ctg cag gcc cct ccg gac gtc acc acg gtg gcc gtg gag gag aca ata 1112
ccc tca ttc acg ggg agg agc cca aac cac tga cccacagact ctgcaccccg 1165
acgccagaga tacctggagc gacggctgct gaaagaggct gtccacctgg cgaaaccacc 1225
ggagcccgga ggcttggggg ctccgccctg ggctggcttc cgtctcctcc agtggaggga 1285
gaggtggggc ccctgctggg gtagagctgg ggacgccacg tgccattccc atgggccagt 1345
gagggcctgg ggcctctgtt ctgctgtggc ctgagctccc cagagtcctg aggaggagcg 1405
ccagttgccc ctcgctcaca gaccacacac ccagccctcc tgggccagcc cagagggccc 1465
ttcagacccc agctgtctgc gcgtctgact cttgtggcct cagcaggaca ggccccgggc 1525
actgcctcac agccaaggct ggactgggtt ggctgcagtg tggtgtttag tggataccac 1585
atcggaagtg attttctaaa ttggatttga attccggtcc tgtcttctat ttgtcatgaa 1645
acagtgtatt tggggagatg ctgtgggagg atgtaaatat cttgtttctc ctcaaaaaaa 1705
aaaaaaaaaa aaaaaaaaa 1724

<210> 2
<211> 283
<212> PRT
<213> Homo sapiens

<400> 2

<210>  3
<211>  1082
<212>  DNA
<213>  Homo sapiens

<220>
<221>  CDS
<222>  (185)..(1012)
<223>

<400>  3
cagaatatca atgctccatt tattcttggt ggaagataaa gaaggctggc tcctgttcct      60
agcgccgaat gtgctcagaa gcgtataggc tgaagaagcg ggcagtgata aattggctca     120
tgagagtgtg tgaggaggtt cagagcccat ggggctcttg gcctgaagtt tcttgatcaa     180
gaaa atg gaa cct ctc cca gga tgg ggg tcg gca ccc tgg agc cag gcc      229
     Met Glu Pro Leu Pro Gly Trp Gly Ser Ala Pro Trp Ser Gln Ala
     1               5                   10                  15
cct aca gac aac acc ttc agg ctg gtg cct tgt gtc ttc ctt ttg aac      277
Pro Thr Asp Asn Thr Phe Arg Leu Val Pro Cys Val Phe Leu Leu Asn
                20                  25                  30
ttg ctg cag cgc atc tct gcc cag ccc tca tgc aga cag gag gag ttc      325
Leu Leu Gln Arg Ile Ser Ala Gln Pro Ser Cys Arg Gln Glu Glu Phe
            35                  40                  45
ctt gtg gga gac gag tgc tgc ccc atg tgc aac cca ggt tac cat gtg      373
Leu Val Gly Asp Glu Cys Cys Pro Met Cys Asn Pro Gly Tyr His Val
        50                  55                  60
aag cag gtc tgc agt gag cat aca ggc aca gtg tgt gcc ccc tgt ccc      421
Lys Gln Val Cys Ser Glu His Thr Gly Thr Val Cys Ala Pro Cys Pro
    65                  70                  75

cca cag aca tat acc gcc cat gca aat ggc ctg agc aag tgt ctg ccc 469
Pro Gln Thr Tyr Thr Ala His Ala Asn Gly Leu Ser Lys Cys Leu Pro
80                  85                  90                  95
tgc gga gtc cgt gat cca gac atg ggc ctg ctg acc tgg cag gag tgc 517
Cys Gly Val Arg Asp Pro Asp Met Gly Leu Leu Thr Trp Gln Glu Cys
                100                 105                 110
tcc agc tgg aag gac act gtg tgc aga tgc atc cca ggc tac ttc tgt 565
Ser Ser Trp Lys Asp Thr Val Cys Arg Cys Ile Pro Gly Tyr Phe Cys
            115                 120                 125
gag aac cag gat ggg agc cac tgt tcc aca tgc ttg cag cac acc acc 613
Glu Asn Gln Asp Gly Ser His Cys Ser Thr Cys Leu Gln His Thr Thr
        130                 135                 140
tgc cct cca ggg cag agg gta gag aag aga ggg act cac gac cag gac 661
Cys Pro Pro Gly Gln Arg Val Glu Lys Arg Gly Thr His Asp Gln Asp
    145                 150                 155
act gta tgt gct gac tgc cta aca ggg acc ttc tca ctt gga ggg act 709
Thr Val Cys Ala Asp Cys Leu Thr Gly Thr Phe Ser Leu Gly Gly Thr
160                 165                 170                 175
cag gag gaa tgc ctg ccc tgg acc aac tgc agt gca ttt caa cag gaa 757
Gln Glu Glu Cys Leu Pro Trp Thr Asn Cys Ser Ala Phe Gln Gln Glu
                180                 185                 190
gta aga cgt ggg acc aac agc aca gac acc acc tgc tcc tcc cag gtc 805
Val Arg Arg Gly Thr Asn Ser Thr Asp Thr Thr Cys Ser Ser Gln Val
            195                 200                 205
gtc tac tac gtt gtg tcc atc ctt ttg cca ctt gtg ata gtg gga gtt 853
Val Tyr Tyr Val Val Ser Ile Leu Leu Pro Leu Val Ile Val Gly Val
        210                 215                 220
ggg ata gct gga ttc ctc atc tgc acg cga aga cac ctg cac acc agc 901
Gly Ile Ala Gly Phe Leu Ile Cys Thr Arg Arg His Leu His Thr Ser
    225                 230                 235
tca gtg gcc aag gag ctg gag cct ttc cag cag gaa caa cag gag aac 949
Ser Val Ala Lys Glu Leu Glu Pro Phe Gln Gln Glu Gln Gln Glu Asn
240                 245                 250                 255
acc atc agg ttt cca gtc acc gag gtt ggg ttt gct gag acc gag gag 997
Thr Ile Arg Phe Pro Val Thr Glu Val Gly Phe Ala Glu Thr Glu Glu
                260                 265                 270
gag aca gcc tcc aac tgaacaaatt ctgggtgaca agacaccgag gagacgttgg 1052
Glu Thr Ala Ser Asn
            275
tgggaagttt ccatatgcta ttgggacatg 1082

<210> 4
<211> 276
<212> PRT
<213> Homo sapiens

<400> 4
Met Glu Pro Leu Pro Gly Trp Gly Ser Ala Pro Trp Ser Gln Ala Pro
1               5                   10                  15

Thr Asp Asn Thr Phe Arg Leu Val Pro Cys Val Phe Leu Leu Asn Leu
            20                  25                  30

Leu Gln Arg Ile Ser Ala Gln Pro Ser Cys Arg Gln Glu Glu Phe Leu
        35                  40                  45

Val Gly Asp Glu Cys Cys Pro Met Cys Asn Pro Gly Tyr His Val Lys
    50                  55                  60

Gln Val Cys Ser Glu His Thr Gly Thr Val Cys Ala Pro Cys Pro Pro
65                  70                  75                  80

Gln Thr Tyr Thr Ala His Ala Asn Gly Leu Ser Lys Cys Leu Pro Cys
                85                  90                  95

Gly Val Arg Asp Pro Asp Met Gly Leu Leu Thr Trp Gln Glu Cys Ser
            100                 105                 110

Ser Trp Lys Asp Thr Val Cys Arg Cys Ile Pro Gly Tyr Phe Cys Glu
        115                 120                 125

Asn Gln Asp Gly Ser His Cys Ser Thr Cys Leu Gln His Thr Thr Cys
    130                 135                 140

Pro Pro Gly Gln Arg Val Glu Lys Arg Gly Thr His Asp Gln Asp Thr
145                 150                 155                 160

Val Cys Ala Asp Cys Leu Thr Gly Thr Phe Ser Leu Gly Gly Thr Gln
                165                 170                 175

Glu Glu Cys Leu Pro Trp Thr Asn Cys Ser Ala Phe Gln Gln Glu Val
            180                 185                 190

Arg Arg Gly Thr Asn Ser Thr Asp Thr Thr Cys Ser Ser Gln Val Val
        195                 200                 205

Tyr Tyr Val Val Ser Ile Leu Leu Pro Leu Val Ile Val Gly Val Gly
    210                 215                 220

Ile Ala Gly Phe Leu Ile Cys Thr Arg Arg His Leu His Thr Ser Ser
225                 230                 235                 240

Val Ala Lys Glu Leu Glu Pro Phe Gln Gln Glu Gln Gln Glu Asn Thr
                245                 250                 255

Ile Arg Phe Pro Val Thr Glu Val Gly Phe Ala Glu Thr Glu Glu Glu
            260                 265                 270

Thr Ala Ser Asn
        275

<210> 5
<211> 1169
<212> DNA
<213> Homo sapiens

<220>
<221> CDS
<222> (49)..(771)
<223>

<400> 5
gaggttgaag gacccaggcg tgtcagccct gctccagaga ccttgggc atg gag gag 57
Met Glu Glu
1
agt gtc gta cgg ccc tca gtg ttt gtg gtg gat gga cag acc gac atc 105
Ser Val Val Arg Pro Ser Val Phe Val Val Asp Gly Gln Thr Asp Ile
    5                   10                  15
cca ttc acg agg ctg gga cga agc cac cgg aga cag tcg tgc agt gtg 153
Pro Phe Thr Arg Leu Gly Arg Ser His Arg Arg Gln Ser Cys Ser Val
20                  25                  30                  35
gcc cgg gtg ggt ctg ggt ctc ttg ctg ttg ctg atg ggg gct ggg ctg 201
Ala Arg Val Gly Leu Gly Leu Leu Leu Leu Leu Met Gly Ala Gly Leu
                40                  45                  50

gcc gtc caa ggc tgg ttc ctc ctg cag ctg cac tgg cgt cta gga gag 249
Ala Val Gln Gly Trp Phe Leu Leu Gln Leu His Trp Arg Leu Gly Glu
            55                  60                  65
atg gtc acc cgc ctg cct gac gga cct gca ggc tcc tgg gag cag ctg 297
Met Val Thr Arg Leu Pro Asp Gly Pro Ala Gly Ser Trp Glu Gln Leu
        70                  75                  80
ata caa gag cga agg tct cac gag gtc aac cca gca gcg cat ctc aca 345
Ile Gln Glu Arg Arg Ser His Glu Val Asn Pro Ala Ala His Leu Thr
    85                  90                  95
ggg gcc aac tcc agc ttg acc ggc agc ggg ggg ccg ctg tta tgg gag 393
Gly Ala Asn Ser Ser Leu Thr Gly Ser Gly Gly Pro Leu Leu Trp Glu
100                 105                 110                 115
act cag ctg ggc ctg gcc ttc ctg agg ggc ctc agc tac cac gat ggg 441
Thr Gln Leu Gly Leu Ala Phe Leu Arg Gly Leu Ser Tyr His Asp Gly
                120                 125                 130
gcc ctt gtg gtc acc aaa gct ggc tac tac tac atc tac tcc aag gtg 489
Ala Leu Val Val Thr Lys Ala Gly Tyr Tyr Tyr Ile Tyr Ser Lys Val
            135                 140                 145
cag ctg ggc ggt gtg ggc tgc ccg ctg ggc ctg gcc agc acc atc acc 537
Gln Leu Gly Gly Val Gly Cys Pro Leu Gly Leu Ala Ser Thr Ile Thr
        150                 155                 160
cac ggc ctc tac aag cgc aca ccc cgc tac ccc gag gag ctg gag ctg 585
His Gly Leu Tyr Lys Arg Thr Pro Arg Tyr Pro Glu Glu Leu Glu Leu
    165                 170                 175
ttg gtc agc cag cag tca ccc tgc gga cgg gcc acc agc agc tcc cgg 633
Leu Val Ser Gln Gln Ser Pro Cys Gly Arg Ala Thr Ser Ser Ser Arg
180                 185                 190                 195
gtc tgg tgg gac agc agc ttc ctg ggt ggt gtg gta cac ctg gag gct 681
Val Trp Trp Asp Ser Ser Phe Leu Gly Gly Val Val His Leu Glu Ala
                200                 205                 210
ggg gag gag gtg gtc gtc cgt gtg ctg gat gaa cgc ctg gtt cga ctg 729
Gly Glu Glu Val Val Val Arg Val Leu Asp Glu Arg Leu Val Arg Leu
            215                 220                 225
cgt gat ggt acc cgg tct tac ttc ggg gct ttc atg gtg tga 771
Arg Asp Gly Thr Arg Ser Tyr Phe Gly Ala Phe Met Val
        230                 235                 240
aggaaggagc gtggtgcatt ggacatgggt ctgacacgtg gagaactcag agggtgcctc 831
aggggaaaga aaactcacga agcagaggct gggcgtggtg gctctcgcct gtaatcccag 891
cactttggga ggccaaggca ggcggatcac ctgaggtcag gagttcgaga ccagcctggc 951
taacatggca aaaccccatc tctactaaaa atacaaaaat tagccggacg tggtggtgcc 1011
tgcctgtaat ccagctactc aggaggctga ggcaggataa ttttgcttaa acccgggagg 1071
cggaggttgc agtgagccga gatcacacca ctgcactcca acctgggaaa cgcagtgaga 1131
ctgtgcctca aaaaaaaaaa aaaaaaaaaa aaaaaaaa 1169

<210> 6
<211> 240
<212> PRT
<213> Homo sapiens

<400> 6
Met Glu Glu Ser Val Val Arg Pro Ser Val Phe Val Val Asp Gly Gln
1               5                   10                  15

Thr Asp Ile Pro Phe Thr Arg Leu Gly Arg Ser His Arg Arg Gln Ser
            20                  25                  30

Cys Ser Val Ala Arg Val Gly Leu Gly Leu Leu Leu Leu Leu Met Gly
        35                  40                  45

Ala Gly Leu Ala Val Gln Gly Trp Phe Leu Leu Gln Leu His Trp Arg
    50                  55                  60

Leu Gly Glu Met Val Thr Arg Leu Pro Asp Gly Pro Ala Gly Ser Trp
65                  70                  75                  80

Glu Gln Leu Ile Gln Glu Arg Arg Ser His Glu Val Asn Pro Ala Ala
                85                  90                  95

His Leu Thr Gly Ala Asn Ser Ser Leu Thr Gly Ser Gly Gly Pro Leu
            100                 105                 110

Leu Trp Glu Thr Gln Leu Gly Leu Ala Phe Leu Arg Gly Leu Ser Tyr
        115                 120                 125

His Asp Gly Ala Leu Val Val Thr Lys Ala Gly Tyr Tyr Tyr Ile Tyr
    130                 135                 140

Ser Lys Val Gln Leu Gly Gly Val Gly Cys Pro Leu Gly Leu Ala Ser
145                 150                 155                 160

Thr Ile Thr His Gly Leu Tyr Lys Arg Thr Pro Arg Tyr Pro Glu Glu
                165                 170                 175

Leu Glu Leu Leu Val Ser Gln Gln Ser Pro Cys Gly Arg Ala Thr Ser
            180                 185                 190

Ser Ser Arg Val Trp Trp Asp Ser Ser Phe Leu Gly Gly Val Val His
        195                 200                 205

Leu Glu Ala Gly Glu Glu Val Val Val Arg Val Leu Asp Glu Arg Leu
    210                 215                 220

Val Arg Leu Arg Asp Gly Thr Arg Ser Tyr Phe Gly Ala Phe Met Val
225                 230                 235                 240

<210>  7
<211>  1894
<212>  DNA
<213>  Murine sp.

<220>
<221>  CDS
<222>  (115)..(834)
<223>

<220>
<221>  Modified_base
<222>  (922)..(925)
<223>  n = a, t, g, or c

<400>  7
tcgacccacg cgtccgagcc cgagcgtgtt gggcaattgt ggtttcctcc ggaagaggag      60
gaactcaggc ttgccaaccc tttccctggg cttcggagcc tcagctgctc tggc atg      117
                                                            Met
                                                            1

gag agt gtg gta cag cct tca gtg ttt gtg gtg gat gga cag acg gac      165
Glu Ser Val Val Gln Pro Ser Val Phe Val Val Asp Gly Gln Thr Asp
            5                   10                  15

atc cca ttc agg cgg ctg gaa cag aac cac cgg aga cgg cgc tgt ggc      213
Ile Pro Phe Arg Arg Leu Glu Gln Asn His Arg Arg Arg Arg Cys Gly
        20                  25                  30

act gtc cag gtc agc ctg gcc ctg gtg ctg ctg cta ggt gct ggg ctg      261
Thr Val Gln Val Ser Leu Ala Leu Val Leu Leu Leu Gly Ala Gly Leu
    35                  40                  45

gcc act cag ggc tgg ttt ctc ctg aga ctg cat caa cgt ctt gga gac 309
Ala Thr Gln Gly Trp Phe Leu Leu Arg Leu His Gln Arg Leu Gly Asp
50                  55                  60                  65
ata gta gct cat ctg cca gat gga ggc aaa ggc tcc tgg gag aag ctg 357
Ile Val Ala His Leu Pro Asp Gly Gly Lys Gly Ser Trp Glu Lys Leu
                70                  75                  80
ata caa gat caa cga tct cac cag gcc aac cca gca gca cat ctt aca 405
Ile Gln Asp Gln Arg Ser His Gln Ala Asn Pro Ala Ala His Leu Thr
            85                  90                  95
gga gcc aac gcc agc ttg ata ggt att ggt gga cct ctg tta tgg gag 453
Gly Ala Asn Ala Ser Leu Ile Gly Ile Gly Gly Pro Leu Leu Trp Glu
        100                 105                 110
aca cga ctt ggc ctg gcc ttc ttg agg ggc ttg acg tat cat gat ggg 501
Thr Arg Leu Gly Leu Ala Phe Leu Arg Gly Leu Thr Tyr His Asp Gly
    115                 120                 125
gcc ctg gtg acc atg gag ccc ggt tac tac tat gtg tac tcc aaa gtg 549
Ala Leu Val Thr Met Glu Pro Gly Tyr Tyr Tyr Val Tyr Ser Lys Val
130                 135                 140                 145
cag ctg agc ggc gtg ggc tgc ccc cag ggg ctg gcc aat ggc ctc ccc 597
Gln Leu Ser Gly Val Gly Cys Pro Gln Gly Leu Ala Asn Gly Leu Pro
                150                 155                 160
atc acc cat gga cta tac aag cgc aca tcc cgc tac ccg aag gag tta 645
Ile Thr His Gly Leu Tyr Lys Arg Thr Ser Arg Tyr Pro Lys Glu Leu
            165                 170                 175
gaa ctg ctg gtc agt cgg cgg tca ccc tgt ggc cgg gcc aac agc tcc 693
Glu Leu Leu Val Ser Arg Arg Ser Pro Cys Gly Arg Ala Asn Ser Ser
        180                 185                 190
cga gtc tgg tgg gac agc agc ttc ctg ggc ggc gtg gta cat ctg gag 741
Arg Val Trp Trp Asp Ser Ser Phe Leu Gly Gly Val Val His Leu Glu
    195                 200                 205
gct ggg gaa gaa gtg gtg gtc cgc gtg cct gga aac cgc ctg gtc aga 789
Ala Gly Glu Glu Val Val Val Arg Val Pro Gly Asn Arg Leu Val Arg
210                 215                 220                 225
cca cgt gac ggc acc agg tcc tat ttc gga gct ttc atg gtc tga 834
Pro Arg Asp Gly Thr Arg Ser Tyr Phe Gly Ala Phe Met Val
                230                 235
aggctgcggt gacaatgtat tttgtggagg gacctctcca ggactcacct caaacccagc 894
aatagggttt gaagtcctcc ctttaagnnn ncctgaactc tgcagtgctc ggggcggtgt 954
agactgctga cctgctttgg gcaatcttca aatcagagac ctggagactt ggggcgtgga 1014
gcccaggagc gagggtcagc tcatttgcct gatattcagg aagaaagaat caagctgggg 1074
tatttatgct tctgatgcaa cactgagatt tcggctttct gggttttgag ctggaggcaa 1134
gaaccttccc agagtgtcat caggaccatg ttggcaggac ttggggctcc agacttgcca 1194
ccacactctg gcctctccca tccatccgct gcattggttt ccagccacca aaacagcact 1254
ggccccctgg ctgcaactgg ccaggtacga gcttctgagc acctacattc ctcagggaca 1314
tcttgatgag atctcagtac tcagtccaat gcgcagcagc gacagacatg ccaggaatgg 1374
ttggtcagaa gggaagggag gaaagggagg aaagaaggga atgcagaaga gaagggggga 1434
aaacaagacc aaaacaaaac agcaacaaca aagcggcagg gaggaggtga cacccttggg 1494
gatactttag tcaacacact tagaacagat tgtgccaggc ctgttggatt cctggagttg 1554
atgggatcgt gggaaggcac aatggggagc aagtgggctt gggttatggc tcagtgggta 1614
aagtgcaatt atggggatct gagtttgaat ccctggtacc catataaaga cacagatgcg 1674
gtgatgggca cttgtgacaa tgagatcatc aatagggaat ggagacagga gggacctctg 1734
gggttcactg gccaggcagt ctagctgaat caaagagctc caagttcagt cgatagctcc 1794
tgaagatgac aactgaggct attctccaaa ccccacacgc aggacacatg cgtaataaat 1854
aaaattttaa aaatattaaa aaaaaaaaaa aaaaaaaaaa 1894

<210> 8
<211> 239
<212> PRT
<213> Murine sp.

<400> 8
Met Glu Ser Val Val Gln Pro Ser Val Phe Val Val Asp Gly Gln Thr
1               5                   10                  15

Asp Ile Pro Phe Arg Arg Leu Glu Gln Asn His Arg Arg Arg Arg Cys
            20                  25                  30

Gly Thr Val Gln Val Ser Leu Ala Leu Val Leu Leu Leu Gly Ala Gly
        35                  40                  45

Leu Ala Thr Gln Gly Trp Phe Leu Leu Arg Leu His Gln Arg Leu Gly
    50                  55                  60

Asp Ile Val Ala His Leu Pro Asp Gly Gly Lys Gly Ser Trp Glu Lys
65                  70                  75                  80

Leu Ile Gln Asp Gln Arg Ser His Gln Ala Asn Pro Ala Ala His Leu
                85                  90                  95

Thr Gly Ala Asn Ala Ser Leu Ile Gly Ile Gly Gly Pro Leu Leu Trp
            100                 105                 110

Glu Thr Arg Leu Gly Leu Ala Phe Leu Arg Gly Leu Thr Tyr His Asp
        115                 120                 125

Gly Ala Leu Val Thr Met Glu Pro Gly Tyr Tyr Tyr Val Tyr Ser Lys
    130                 135                 140

Val Gln Leu Ser Gly Val Gly Cys Pro Gln Gly Leu Ala Asn Gly Leu
145                 150                 155                 160

Pro Ile Thr His Gly Leu Tyr Lys Arg Thr Ser Arg Tyr Pro Lys Glu
                165                 170                 175

Leu Glu Leu Leu Val Ser Arg Arg Ser Pro Cys Gly Arg Ala Asn Ser
            180                 185                 190

Ser Arg Val Trp Trp Asp Ser Ser Phe Leu Gly Gly Val Val His Leu
        195                 200                 205

Glu Ala Gly Glu Glu Val Val Val Arg Val Pro Gly Asn Arg Leu Val
    210                 215                 220

Arg Pro Arg Asp Gly Thr Arg Ser Tyr Phe Gly Ala Phe Met Val
225                 230                 235

<210> 9
<211> 193
<212> PRT
<213> Homo sapiens

<400> 9

<210>  10
<211>  277
<212>  PRT
<213>  Homo sapiens

<400>  10
Met Glu Pro Pro Gly Asp Trp Gly Pro Pro Pro Trp Arg Ser Thr Pro
1               5                   10                  15

Arg Thr Asp Val Leu Arg Leu Val Leu Tyr Leu Thr Phe Leu Gly Ala
            20                  25                  30

Pro Cys Tyr Ala Pro Ala Leu Pro Ser Cys Lys Glu Asp Glu Tyr Pro
        35                  40                  45

Val Gly Ser Glu Cys Cys Pro Lys Cys Ser Pro Gly Tyr Arg Val Lys
    50                  55                  60

Glu Ala Cys Gly Glu Leu Thr Gly Thr Val Cys Glu Pro Cys Pro Pro
65                  70                  75                  80

Gly Thr Tyr Ile Ala His Leu Asn Gly Leu Ser Lys Cys Leu Gln Cys
                85                  90                  95

Gln Met Cys Asp Pro Ala Met Gly Leu Arg Ala Ser Arg Asn Cys Ser
            100                 105                 110

Arg Thr Glu Asn Ala Val Cys Gly Cys Ser Pro Gly His Phe Cys Ile
        115                 120                 125

Val Gln Asp Gly Asp His Cys Ala Ala Cys Arg Ala Tyr Ala Thr Ser
    130                 135                 140

Ser Pro Gly Gln Arg Val Gln Lys Gly Gly Thr Glu Ser Gln Asp Thr
145                 150                 155                 160

Leu Cys Gln Asn Cys Pro Pro Gly Thr Phe Ser Pro Asn Gly Thr Leu
                165                 170                 175

Glu Glu Cys Gln His Gln Thr Lys Cys Ser Trp Leu Val Thr Lys Ala
            180                 185                 190

Gly Ala Gly Thr Ser Ser Ser His Trp Val Trp Trp Phe Leu Ser Gly
        195                 200                 205

Ser Leu Val Ile Val Ile Val Cys Ser Thr Val Gly Leu Ile Ile Cys
    210                 215                 220

Val Lys Arg Arg Lys Pro Arg Gly Asp Val Val Lys Val Ile Val Ser
225                 230                 235                 240

Val Gln Val Leu Ile Leu Leu Pro Leu Ser Leu Pro Pro Pro Pro Ser
                245                 250                 255

His Leu Pro Ser Pro Arg Trp Gly Trp Cys Phe Trp Cys Thr Trp Trp
            260                 265                 270

Gly Leu Pro Val Leu
        275

<210>  11
<211>  186
<212>  PRT
<213>  Homo sapiens

<400>  11
Met Glu Pro Pro Gly Asp Trp Gly Pro Pro Pro Trp Arg Ser Thr Pro
1               5                   10                  15

Arg Thr Asp Val Ser Arg Leu Val Leu Tyr Leu Thr Phe Leu Gly Ala
            20                  25                  30

Pro Cys Tyr Ala Pro Ala Leu Pro Ser Cys Lys Glu Asp Glu Tyr Pro
        35                  40                  45

Val Gly Ser Glu Cys Cys Pro Lys Cys Ser Pro Gly Tyr Arg Val Lys
    50                  55                  60

Glu Ala Cys Gly Glu Leu Thr Gly Thr Val Cys Glu Pro Cys Pro Pro
65                  70                  75                  80

Gly Thr Tyr Ile Ala His Leu Asn Gly Leu Ser Lys Cys Leu Gln Cys
                85                  90                  95

Gln Met Cys Asp Pro Ala Met Gly Leu Arg Ala Ser Arg Asn Cys Ser
            100                 105                 110

Arg Thr Glu Asn Ala Val Cys Gly Cys Ser Pro Gly His Phe Cys Ile
        115                 120                 125

Val Gln Asp Gly Asp His Cys Ala Ala Cys Arg Ala Tyr Ala Thr Ser
    130                 135                 140

Ser Pro Gly Gln Arg Val Gln Lys Gly Gly Thr Glu Ser Gln Asp Thr
145                 150                 155                 160

Leu Cys Gln Asn Cys Pro Pro Gly Thr Phe Ser Pro Asn Gly Thr Leu
                165                 170                 175

Glu Glu Cys Gln His Gln Thr Lys Lys Ala
            180                 185

<210> 12
<211> 1206
<212> DNA
<213> Herpes simplex virus

<220>
<221> CDS
<222> (1)..(1185)
<223>

<400> 12
   atg ggg ggg gct gcc gcc agg ttg ggg gcc gtg att ttg ttt gtc gtc 48
   Met Gly Gly Ala Ala Ala Arg Leu Gly Ala Val Ile Leu Phe Val Val
   1 5 10 15
   ata gtg ggc ctc cat ggg gtc cgc ggc aaa tat gcc ttg gcg gat gcc 96
   Ile Val Gly Leu His Gly Val Arg Gly Lys Tyr Ala Leu Ala Asp Ala
   20 25 30
   tct ctc aag atg gcc gac ccc aat cgc ttt cgc ggc aaa gac ctt ccg 144
   Ser Leu Lys Met Ala Asp Pro Asn Arg Phe Arg Gly Lys Asp Leu Pro
   35 40 45
   gtc ctg gac cag ctg acc gac cct ccg ggg gtc cgg cgc gtg tac cac 192
   Val Leu Asp Gln Leu Thr Asp Pro Pro Gly Val Arg Arg Val Tyr His
   50 55 60
   atc cag gcg ggc cta ccg gac ccg ttc cag ccc ccc agc ctc ccg atc 240
   Ile Gln Ala Gly Leu Pro Asp Pro Phe Gln Pro Pro Ser Leu Pro Ile
   65 70 75 80
   acg gtt tac tac gcc gtg ttg gag cgc gcc tgc cgc agc gtg ctc cta 288
   Thr Val Tyr Tyr Ala Val Leu Glu Arg Ala Cys Arg Ser Val Leu Leu
   85 90 95
   aac gca ccg tcg gag gcc ccc cag att gtc cgc ggg gcc tcc gaa gac 336
   Asn Ala Pro Ser Glu Ala Pro Gln Ile Val Arg Gly Ala Ser Glu Asp
   100 105 110
   gtc cgg aaa caa ccc tac aac ctg acc atc gct tgg ttt cgg atg gga 384
   Val Arg Lys Gln Pro Tyr Asn Leu Thr Ile Ala Trp Phe Arg Met Gly
   115 120 125
   ggc aac tgt gct atc ccc atc acg gtc atg gag tac acc gaa tgc tcc 432
   Gly Asn Cys Ala Ile Pro Ile Thr Val Met Glu Tyr Thr Glu Cys Ser
   130 135 140
   tac aac aag tct ctg ggg gcc tgt ccc atc cga acg cag ccc cgc tgg 480
   Tyr Asn Lys Ser Leu Gly Ala Cys Pro Ile Arg Thr Gln Pro Arg Trp
   145 150 155 160
   aac tac tat gac agc ttc agc gcc gtc agc gag gat aac ctg ggg ttc 528
   Asn Tyr Tyr Asp Ser Phe Ser Ala Val Ser Glu Asp Asn Leu Gly Phe
   165 170 175
   ctg atg cac gcc ccc gcg ttt gag acc gcc ggc acg tac ctg cgg ctc 576
   Leu Met His Ala Pro Ala Phe Glu Thr Ala Gly Thr Tyr Leu Arg Leu
   180 185 190
   gtg aag ata aac gac tgg acg gag att aca cag ttt atc ctg gag cac 624
   Val Lys Ile Asn Asp Trp Thr Glu Ile Thr Gln Phe Ile Leu Glu His
   195 200 205
   cga gcc aag ggc tcc tgt aag tac gcc ctc ccg ctg cgc atc ccc ccg 672
   Arg Ala Lys Gly Ser Cys Lys Tyr Ala Leu Pro Leu Arg Ile Pro Pro
   210 215 220
   tca gcc tgc ctg tcc ccc cag gcc tac cag cag ggg gtg acg gtg gac 720
   Ser Ala Cys Leu Ser Pro Gln Ala Tyr Gln Gln Gly Val Thr Val Asp
   225 230 235 240
   agc atc ggg atg ctg ccc cgc ttc atc ccc gag aac cag cgc acc gtc 768
   Ser Ile Gly Met Leu Pro Arg Phe Ile Pro Glu Asn Gln Arg Thr Val
   245 250 255
   gcc gta tac agc ttg aag atc gcc ggg tgg cac ggg ccc aag gcc cca 816
   Ala Val Tyr Ser Leu Lys Ile Ala Gly Trp His Gly Pro Lys Ala Pro
   260 265 270
   tac acg agc acc ctg ctg ccc ccg gag ctg tcc gag acc ccc aac gcc 864
   Tyr Thr Ser Thr Leu Leu Pro Pro Glu Leu Ser Glu Thr Pro Asn Ala
   275 280 285
   acg cag cca gaa ctc gcc ccg gaa gac ccc gag gat tcg gcc ctc ttg 912
   Thr Gln Pro Glu Leu Ala Pro Glu Asp Pro Glu Asp Ser Ala Leu Leu
   290 295 300
   gag gac ccc gtg ggg acg gtg gcg ccg caa atc cca cca aac tgg cac 960
   Glu Asp Pro Val Gly Thr Val Ala Pro Gln Ile Pro Pro Asn Trp His
   305 310 315 320
   ata ccg tcg atc cag gac gcc gcg acg cct tac cat ccc ccg gcc acc 1008
   Ile Pro Ser Ile Gln Asp Ala Ala Thr Pro Tyr His Pro Pro Ala Thr
   325 330 335
   ccg aac aac atg ggc ctg atc gcc ggc gcg gtg ggc ggc agt ctc ctg 1056
   Pro Asn Asn Met Gly Leu Ile Ala Gly Ala Val Gly Gly Ser Leu Leu
   340 345 350
   gca gcc ctg gtc att tgc gga att gtg tac tgg atg cgc cgc cgc act 1104
   Ala Ala Leu Val Ile Cys Gly Ile Val Tyr Trp Met Arg Arg Arg Thr
   355 360 365
   caa aaa gcc cca aag cgc ata cgc ctc ccc cac atc cgg gaa gac gac 1152
   Gln Lys Ala Pro Lys Arg Ile Arg Leu Pro His Ile Arg Glu Asp Asp
   370 375 380
   cag ccg tcc tcg cac cag ccc ttg ttt tac tag ataccccccc ttaatgggtg 1205
   Gln Pro Ser Ser His Gln Pro Leu Phe Tyr
   385 390
   c 1206

<210> 13
<211> 394
<212> PRT
<213> Herpes simplex virus

<400> 13
Met Gly Gly Ala Ala Ala Arg Leu Gly Ala Val Ile Leu Phe Val Val
1               5                   10                  15

Ile Val Gly Leu His Gly Val Arg Gly Lys Tyr Ala Leu Ala Asp Ala
            20                  25                  30

Ser Leu Lys Met Ala Asp Pro Asn Arg Phe Arg Gly Lys Asp Leu Pro
        35                  40                  45

Val Leu Asp Gln Leu Thr Asp Pro Pro Gly Val Arg Arg Val Tyr His
    50                  55                  60

Ile Gln Ala Gly Leu Pro Asp Pro Phe Gln Pro Pro Ser Leu Pro Ile
65                  70                  75                  80

Thr Val Tyr Tyr Ala Val Leu Glu Arg Ala Cys Arg Ser Val Leu Leu
                85                  90                  95

Asn Ala Pro Ser Glu Ala Pro Gln Ile Val Arg Gly Ala Ser Glu Asp
            100                 105                 110

Val Arg Lys Gln Pro Tyr Asn Leu Thr Ile Ala Trp Phe Arg Met Gly
        115                 120                 125

Gly Asn Cys Ala Ile Pro Ile Thr Val Met Glu Tyr Thr Glu Cys Ser
    130                 135                 140

Tyr Asn Lys Ser Leu Gly Ala Cys Pro Ile Arg Thr Gln Pro Arg Trp
145                 150                 155                 160

Asn Tyr Tyr Asp Ser Phe Ser Ala Val Ser Glu Asp Asn Leu Gly Phe
                165                 170                 175

Leu Met His Ala Pro Ala Phe Glu Thr Ala Gly Thr Tyr Leu Arg Leu
            180                 185                 190

Val Lys Ile Asn Asp Trp Thr Glu Ile Thr Gln Phe Ile Leu Glu His
        195                 200                 205

Arg Ala Lys Gly Ser Cys Lys Tyr Ala Leu Pro Leu Arg Ile Pro Pro
    210                 215                 220

Ser Ala Cys Leu Ser Pro Gln Ala Tyr Gln Gln Gly Val Thr Val Asp
225                 230                 235                 240

Ser Ile Gly Met Leu Pro Arg Phe Ile Pro Glu Asn Gln Arg Thr Val
                245                 250                 255

Ala Val Tyr Ser Leu Lys Ile Ala Gly Trp His Gly Pro Lys Ala Pro
            260                 265                 270

Tyr Thr Ser Thr Leu Leu Pro Pro Glu Leu Ser Glu Thr Pro Asn Ala
        275                 280                 285

Thr Gln Pro Glu Leu Ala Pro Glu Asp Pro Glu Asp Ser Ala Leu Leu
    290                 295                 300

Glu Asp Pro Val Gly Thr Val Ala Pro Gln Ile Pro Pro Asn Trp His
305                 310                 315                 320

Ile Pro Ser Ile Gln Asp Ala Ala Thr Pro Tyr His Pro Pro Ala Thr
                325                 330                 335

Pro Asn Asn Met Gly Leu Ile Ala Gly Ala Val Gly Gly Ser Leu Leu
            340                 345                 350

Ala Ala Leu Val Ile Cys Gly Ile Val Tyr Trp Met Arg Arg Arg Thr
        355                 360                 365

Gln Lys Ala Pro Lys Arg Ile Arg Leu Pro His Ile Arg Glu Asp Asp
    370                 375                 380

Gln Pro Ser Ser His Gln Pro Leu Phe Tyr
385                 390

<210>  14
<211>  5033
<212>  DNA
<213>  Homo sapiens

<220>
<221>  CDS
<222>  (2583)..(2681)
<223>

<220>
<221> CDS
<222> (2768)..(2873)
<223>

<220>
<221> CDS
<222> (3121)..(3530)
<223>

<400> 14
tattggccat aacccttatg atgggtcagg aagggatcac ccctttctgc ccctacagaa 60
ataatagcta agactagtaa agcataaaag gcaaaggggc aggtcctcaa gtagagaaga 120
acaggagaaa tagctcatac acacccagaa tgttacttac atgtccctcc atgttacacc 180
aagacccctc agggaccttg tgcctgggga gagaagtggt ctgccccatg caacagtggg 240
ctttaccccg ggtcaccacc agccccagct ccaacccctc taacactctc caagtaaaat 300
cacatcagta gcagtaataa tatttgaggt gacaagttgg tattatctca aacttaggaa 360
aagtgaataa agtcatcttt agaaactgct ttttttaaac cttgtaactt gcaagctaag 420
tgaaaatggg ctcatgtatg agaatgttcg tgttagacat tttttgtgtt agacaaaaac 480
tagaaacaaa ccaaatcccc atcaacagaa tatattagaa tatattgata caatagaata 540
ttacatcata atttttttta aaaacattac tgatacatac aaccacgtat atgaatctca 600
caaacataat gctgactgaa agaagtcaaa cagaaatgag tacattctgt gtgatttcat 660
ttatatgatg ccccaaacca ggaggaaata atctatggtg ataaaagtga gagagtggtt 720
ggttatcttt ggagggtatc agcagggagg gggcatgagg gaacctgctg gggacctgaa 780
aatacgtgga gctgggtggt ggctacatac agatggaaaa attcatcagc tgtacactta 840
agaggtgtcc acctcatacc taagttacat atcaataaaa aggaaaaaaa ttttggaaac 900
tttttttttt ttttttgaga cagagtcttg ctctgtcccc caggctggaa tacagtggtg 960
cgatcttgac tcactgcagc ctccgcctcc caggttcaaa taattctcca gcctcagcct 1020
cccgagtagc tgggactgca gatgcgcacc agcacgcctg gctaattttt gtatttatta 1080
tagagatggg gtttcaccat gttggccagc tggtctcaaa ctcctgacct caagtaatcc 1140
gcccacctca gactcccaaa gtgccaggat tacaggtgtg agccactgca ccaggcctgg 1200
aacaatttta aaataatgta ttggctctgc aaatgcagct tcagaacaag tcccttagct 1260
gtccccaccc caccctaagt caccaccctt aagcctcacc catgtggaat tctgaaactt 1320
cctttgtaga aaactttgga aggtgtctgc cacattgatc ctggaatgtg tgtttatttg 1380
gggttatata aatctgttct gtggaagcca cctgaagtca ggaagagatg gagggcatcc 1440
ttcaggagtg agatgagacc tcatcatact tgactgtcca gcatcatctc tgagtaaggg 1500
gaccaaaaaa tttatcttcc aaactaggac actttcaaga gtggaagggg gatccattaa 1560
tattttcacc tggacaagag gcaaacacca gaatgtcccc gatgaagggg atatataatg 1620
gaccttcttg atgtgaaacc tgccagatgg gctggaaagt ccgtatactg ggacaagtat 1680
gatttgagtt gtttgggaca aggacagggg tacaagagaa ggaaatgggc aaagagagaa 1740
gcctgtactc agccaagggt gcagagatgt tatatatgat tgctcttcag ggaaccgggc 1800
ctccagctca caccccagct gctcaaccgc ctcctctctg aattgactgt cccttctttg 1860
gaactctagg cctgacccca ctccctggcc ctcccagccc acgattcccc tgacccgact 1920
ccctttccca gaactcagtc gcctgaaccc ccagcctgtg gttctctcct aggcctcagc 1980
ctttcctgcc tttgactgaa acagcagtat cttctaagcc ctgggggctt ccccgggccc 2040
cagccccgac ctagaacccg cccgctgcct gccacgctgc cactgccgct tcctctataa 2100
agggacctga gcgtccgggc ccaggggctc cgcacagcag gtgaggctct cctgccccat 2160
ctccttgggc tgcccgtgct tcgtgctttg gactaccgcc ccgcagtgtc ctgccctctg 2220
cctgggcctc ggtccctcct gcacctgctg cctggatccc cggcctgcct gggcctgggc 2280
cttggtgggt ttggttttgg tttccttctc tgtctctgac tctccatctg tcagtctcat 2340
tgtctctgtc acacattctc tgtttctgcc atgattcctc tctgttccct tcctgtctct 2400
ctctgtctcc ctctgctcac cttggggttt ctctgactgc atcttgtccc cttctctgtc 2460
gatctctctc tcgggggtcg gggggtgctg tctcccaggg cgggaggtct gtcttccgcc 2520
gcgtgccccg ccccgctcac tgtctctctc tctctctctc tctttctctg caggttctcc 2580
cc atg aca cca cct gaa cgt ctc ttc ctc cca agg gtg tgt ggc acc 2627
Met Thr Pro Pro Glu Arg Leu Phe Leu Pro Arg Val Cys Gly Thr
1               5                   10                  15
acc cta cac ctc ctc ctt ctg ggg ctg ctg ctg gtt ctg ctg cct ggg 2675
Thr Leu His Leu Leu Leu Leu Gly Leu Leu Leu Val Leu Leu Pro Gly
                20                  25                  30
gcc cag gtgaggcagc aggagaatgg gggctgctgg ggtggctcag ccaaaccttg 2731
Ala Gln

agccctagag cccccctcaa ctctgttctc ccctag ggg ctc cct ggt gtt ggc     2785
                                        Gly Leu Pro Gly Val Gly
                                                    35
ctc aca cct tca gct gcc cag act gcc cgt cag cac ccc aag atg cat     2833
Leu Thr Pro Ser Ala Ala Gln Thr Ala Arg Gln His Pro Lys Met His
40                  45                  50                  55
ctt gcc cac agc acc ctc aaa cct gct gct cac ctc att g gtaaacatcc    2883
Leu Ala His Ser Thr Leu Lys Pro Ala Ala His Leu Ile
                60                  65
acctgacctc ccagacatgt ccccaccagc tctcctccta cccctgcctc aggaacccaa   2943
gcatccaccc ctctccccca acttccccca cgctaaaaaa aacagaggga gcccactcct   3003
atgcctcccc ctgccatccc ccaggaactc agttgttcag tgcccacttc ctcagggatt   3063
gagacctctg atccagaccc ctgatctccc acccccatcc cctatggctc ttcctag      3120
ga  gac ccc agc aag cag aac tca ctg ctc tgg aga gca aac acg gac     3167
Gly Asp Pro Ser Lys Gln Asn Ser Leu Leu Trp Arg Ala Asn Thr Asp
    70                  75                  80
cgt gcc ttc ctc cag gat ggt ttc tcc ttg agc aac aat tct ctc ctg     3215
Arg Ala Phe Leu Gln Asp Gly Phe Ser Leu Ser Asn Asn Ser Leu Leu
85                  90                  95                  100
gtc ccc acc agt ggc atc tac ttc gtc tac tcc cag gtg gtc ttc tct     3263
Val Pro Thr Ser Gly Ile Tyr Phe Val Tyr Ser Gln Val Val Phe Ser
                105                 110                 115
ggg aaa gcc tac tct ccc aag gcc acc tcc tcc cca ctc tac ctg gcc     3311
Gly Lys Ala Tyr Ser Pro Lys Ala Thr Ser Ser Pro Leu Tyr Leu Ala
            120                 125                 130
cat gag gtc cag ctc ttc tcc tcc cag tac ccc ttc cat gtg cct ctc     3359
His Glu Val Gln Leu Phe Ser Ser Gln Tyr Pro Phe His Val Pro Leu
        135                 140                 145
ctc agc tcc cag aag atg gtg tat cca ggg ctg cag gaa ccc tgg ctg     3407
Leu Ser Ser Gln Lys Met Val Tyr Pro Gly Leu Gln Glu Pro Trp Leu
    150                 155                 160
cac tcg atg tac cac ggg gct gcg ttc cag ctc acc cag gga gac cag     3455
His Ser Met Tyr His Gly Ala Ala Phe Gln Leu Thr Gln Gly Asp Gln
165                 170                 175                 180
cta tcc acc cac aca gat ggc atc ccc cac cta gtc ctc agc cct agt     3503
Leu Ser Thr His Thr Asp Gly Ile Pro His Leu Val Leu Ser Pro Ser
                185                 190                 195
act gtc ttc ttt gga gcc ttc gct ctg tagaacttgg aaaaatccag           3550
Thr Val Phe Phe Gly Ala Phe Ala Leu
            200                 205
aaagaaaaaa taattgattt caagaccttc tccccattct gcctccattc tgaccatttc   3610
aggggtcgtc accacctctc ctttggccat tccaacagct caagtcttcc ctgatcaagt   3670
caccggagct ttcaaagaag gaattctagg catcccaggg gaccacacct ccctgaacca   3730
tccctgatgt ctgtctggct gaggatttca agcctgccta ggaattccca gcccaaagct   3790
gttggtctgt cccaccagct aggtggggcc tagatccaca cacagaggaa gagcaggcac   3850
atggaggagc ttgggggatg actagaggca gggaggggac tatttatgaa ggcaaaaaaa   3910
ttaaattatt tatttatgga ggatggagag aggggaataa tagaagaaca tccaaggaga   3970
aacagagaca ggcccaagag atgaagagtg agagggcatg cgcacaaggc tgaccaagag   4030
agaaagaagt aggcatgagg gatcacaggg ccccagaagg cagggaaagg ctctgaaagc   4090
cagctgccga ccagagcccc acacggaggc atctgcaccc tcgatgaagc ccaataaacc   4150
tcttttctct gaaatgctgt ctgcttgtgt gtgtgtgtct gggagtgaga acttcccagt   4210
ctatctaagg aatggaggga gggacagagg gctcaaaggg agcaagagct gtggggagaa   4270
caaaaggata agggctcaga gagcttcagg gatatgtgat ggactcacca ggtgaggccg   4330
ccagactgct gcaggggaag caaaggagaa gctgagaaga tgaaggaaaa gtcagggtct   4390
ggaggggcgg gggtcaggga gctcctggga gatatggcca catgtagcgg ctctgaggaa   4450
tgggttacag gagacctctg gggagatgtg accacagcaa tgggtaggag aatgtccagg   4510
gctatggaag tcgagtatgg ggaccccccc ttaacgaaga cagggccatg tagagggccc   4570
cagggagtga aagagcctcc aggacctcca ggtatggaat acaggggacg tttaagaaga   4630
tatggccaca cactggggcc ctgagaagtg agagcttcat gaaaaaaatc agggacccca   4690
gagttccttg gaagccaaga ctgaaaccag cattatgagt ctccgggtca gaatgaaaga   4750
agaaggcctg ccccagtggg gtctgtgaat tcccgggggt gatttcactc cccggggctg    4810

tcccaggctt gtccctgcta cccccaccca gcctttcctg aggcctcaag cctgccacca 4870
agcccccagc tccttctccc cgcagggacc caaacacagg cctcaggact caacacagct 4930
tttccctcca accccgtttt ctctccctca aggactcagc tttctgaagc ccctcccagt 4990
tctagttcta tctttttcct gcatcctgtc tggaagttag aag 5033

<210> 15
<211> 205
<212> PRT
<213> Homo sapiens;

<400> 15
Met Thr Pro Pro Glu Arg Leu Phe Leu Pro Arg Val Cys Gly Thr Thr
1               5                   10                  15

Leu His Leu Leu Leu Leu Gly Leu Leu Leu Val Leu Leu Pro Gly Ala
            20                  25                  30

Gln Gly Leu Pro Gly Val Gly Leu Thr Pro Ser Ala Ala Gln Thr Ala
        35                  40                  45

Arg Gln His Pro Lys Met His Leu Ala His Ser Thr Leu Lys Pro Ala
    50                  55                  60

Ala His Leu Ile Gly Asp Pro Ser Lys Gln Asn Ser Leu Leu Trp Arg
65                  70                  75                  80

Ala Asn Thr Asp Arg Ala Phe Leu Gln Asp Gly Phe Ser Leu Ser Asn
                85                  90                  95

Asn Ser Leu Leu Val Pro Thr Ser Gly Ile Tyr Phe Val Tyr Ser Gln
            100                 105                 110

Val Val Phe Ser Gly Lys Ala Tyr Ser Pro Lys Ala Thr Ser Ser Pro
        115                 120                 125

Leu Tyr Leu Ala His Glu Val Gln Leu Phe Ser Ser Gln Tyr Pro Phe
    130                 135                 140

His Val Pro Leu Leu Ser Ser Gln Lys Met Val Tyr Pro Gly Leu Gln
145                 150                 155                 160

Glu Pro Trp Leu His Ser Met Tyr His Gly Ala Ala Phe Gln Leu Thr
                165                 170                 175

Gln Gly Asp Gln Leu Ser Thr His Thr Asp Gly Ile Pro His Leu Val
            180                 185                 190

Leu Ser Pro Ser Thr Val Phe Phe Gly Ala Phe Ala Leu
        195                 200                 205

<210> 16
<211> 2105
<212> DNA
<213> Homo sapiens

<220>
<221> CDS
<222> (169)..(1476)
<223>

<400> 16
gccctggagg cccggcctgg ccgctcccgg ccctggggtg cacatcggcc ctgagtcccg 60

tcccaggctc tgggctcggg cagccgccgc caccgctgcc caggacgtcg ggcctcctgc 120
cttcctccca ggcccccacg ttgctggccg cctggccgag tggccgcc atg ctc ctg 177
Met Leu Leu
1
cct tgg gcc acc tct gcc ccc ggc ctg gcc tgg ggg cct ctg gtg ctg 225
Pro Trp Ala Thr Ser Ala Pro Gly Leu Ala Trp Gly Pro Leu Val Leu
    5                   10                  15
ggc ctc ttc ggg ctc ctg gca gca tcg cag ccc cag gcg gtg cct cca 273
Gly Leu Phe Gly Leu Leu Ala Ala Ser Gln Pro Gln Ala Val Pro Pro
20                  25                  30                  35
tat gcg tcg gag aac cag acc tgc agg gac cag gaa aag gaa tac tat 321
Tyr Ala Ser Glu Asn Gln Thr Cys Arg Asp Gln Glu Lys Glu Tyr Tyr
                40                  45                  50
gag ccc cag cac cgc atc tgc tgc tcc cgc tgc ccg cca ggc acc tat 369
Glu Pro Gln His Arg Ile Cys Cys Ser Arg Cys Pro Pro Gly Thr Tyr
            55                  60                  65
gtc tca gct aaa tgt agc cgc atc cgg gac aca gtt tgt gcc aca tgt 417
Val Ser Ala Lys Cys Ser Arg Ile Arg Asp Thr Val Cys Ala Thr Cys
        70                  75                  80
gcc gag aat tcc tac aac gag cac tgg aac tac ctg acc atc tgc cag 465
Ala Glu Asn Ser Tyr Asn Glu His Trp Asn Tyr Leu Thr Ile Cys Gln
    85                  90                  95
ctg tgc cgc ccc tgt gac cca gtg atg ggc ctc gag gag att gcc ccc 513
Leu Cys Arg Pro Cys Asp Pro Val Met Gly Leu Glu Glu Ile Ala Pro
100                 105                 110                 115
tgc aca agc aaa cgg aag acc cag tgc cgc tgc cag ccg gga atg ttc 561
Cys Thr Ser Lys Arg Lys Thr Gln Cys Arg Cys Gln Pro Gly Met Phe
                120                 125                 130
tgt gct gcc tgg gcc ctc gag tgt aca cac tgc gag cta ctt tct gac 609
Cys Ala Ala Trp Ala Leu Glu Cys Thr His Cys Glu Leu Leu Ser Asp
            135                 140                 145
tgc ccg cct ggc act gaa gcc gag ctc aaa gat gaa gtt ggg aag ggt 657
Cys Pro Pro Gly Thr Glu Ala Glu Leu Lys Asp Glu Val Gly Lys Gly
        150                 155                 160
aac aac cac tgc gtc ccc tgc aag gcc ggg cac ttc cag aat acc tcc 705
Asn Asn His Cys Val Pro Cys Lys Ala Gly His Phe Gln Asn Thr Ser
    165                 170                 175
tcc ccc agc gcc cgc tgc cag ccc cac acc agg tgt gag aac caa ggt 753
Ser Pro Ser Ala Arg Cys Gln Pro His Thr Arg Cys Glu Asn Gln Gly
180                 185                 190                 195
ctg gtg gag gca gct cca ggc act gcc cag tcc gac aca acc tgc aaa 801
Leu Val Glu Ala Ala Pro Gly Thr Ala Gln Ser Asp Thr Thr Cys Lys
                200                 205                 210
aat cca tta gag cca ctg ccc cca gag atg tca gga acc atg ctg atg 849
Asn Pro Leu Glu Pro Leu Pro Pro Glu Met Ser Gly Thr Met Leu Met
            215                 220                 225
ctg gcc gtt ctg ctg cca ctg gcc ttc ttt ctg ctc ctt gcc acc gtc 897
Leu Ala Val Leu Leu Pro Leu Ala Phe Phe Leu Leu Leu Ala Thr Val
        230                 235                 240
ttc tcc tgc atc tgg aag agc cac cct tct ctc tgc agg aaa ctg gga 945
Phe Ser Cys Ile Trp Lys Ser His Pro Ser Leu Cys Arg Lys Leu Gly
    245                 250                 255
tcg ctg ctc aag agg cgt ccg cag gga gag gga ccc aat cct gta gct 993
Ser Leu Leu Lys Arg Arg Pro Gln Gly Glu Gly Pro Asn Pro Val Ala
260                 265                 270                 275
gga agc tgg gag cct ccg aag gcc cat cca tac ttc cct gac ttg gta 1041
Gly Ser Trp Glu Pro Pro Lys Ala His Pro Tyr Phe Pro Asp Leu Val
                280                 285                 290
cag cca ctg cta ccc att tct gga gat gtt tcc cca gta tcc act ggg 1089
Gln Pro Leu Leu Pro Ile Ser Gly Asp Val Ser Pro Val Ser Thr Gly
            295                 300                 305
ctc ccc gca gcc cca gtt ttg gag gca ggg gtg ccg caa cag cag agt 1137

Leu Pro Ala Ala Pro Val Leu Glu Ala Gly Val Pro Gln Gln Gln Ser
        310                 315                 320
cct ctg gac ctg acc agg gag ccg cag ttg gaa ccc ggg gag cag agc      1185
Pro Leu Asp Leu Thr Arg Glu Pro Gln Leu Glu Pro Gly Glu Gln Ser
    325                 330                 335
cag gtg gcc cac ggt acc aat ggc att cat gtc acc ggc ggg tct atg      1233
Gln Val Ala His Gly Thr Asn Gly Ile His Val Thr Gly Gly Ser Met
340                 345                 350                 355
act atc act ggc aac atc tac atc tac aat gga cca gta ctg ggg gga      1281
Thr Ile Thr Gly Asn Ile Tyr Ile Tyr Asn Gly Pro Val Leu Gly Gly
                360                 365                 370
cca ccg ggt cct gga gac ctc cca gct acc ccc gaa cct cca tac ccc      1329
Pro Pro Gly Pro Gly Asp Leu Pro Ala Thr Pro Glu Pro Pro Tyr Pro
            375                 380                 385
att ccc gaa gag ggg gac cct ggc cct ccc ggg ctc tct aca ccc cac      1377
Ile Pro Glu Glu Gly Asp Pro Gly Pro Pro Gly Leu Ser Thr Pro His
        390                 395                 400
cag gaa gat ggc aag gct tgg cac cta gcg gag aca gag cac tgt ggt      1425
Gln Glu Asp Gly Lys Ala Trp His Leu Ala Glu Thr Glu His Cys Gly
    405                 410                 415
gcc aca ccc tct aac agg ggc cca agg aac caa ttt atc acc cat gac      1473
Ala Thr Pro Ser Asn Arg Gly Pro Arg Asn Gln Phe Ile Thr His Asp
420                 425                 430                 435
tga ctgagtctga gaaaaggcag aagaaggggg gcacaagggc accttctccc           1526
ttgaggctgc cctgcccacg tgggattcac aggggcctga gtagggcccg gggaagcaga    1586
gccctaaggg attaaggctc agacacctct gagagcaggt gggcactggc tgggtacggt    1646
gccctccaca ggactctccc tactgcctga gcaaacctga ggcctcccgg cagacccacc    1706
caccccctgg ggctgctcag cctcaggcac ggacagggca catgatacca actgctgccc   1766
actacggcac gccgcaccgg agcacggcac cgagggagcc gccacacggt cacctgcaag    1826
gacgtcacgg gcccctctaa aggattcgtg gtgctcatcc ccaagcttca gagacccttt    1886
ggggttccac acttcacgtg gactgaggta gaccctgcat gaagatgaaa ttatagggag    1946
gacgctcctt ccctcccctc ctagaggaga ggaaagggag tcattaacaa ctagggggtt   2006
gggtaggatt cctaggtatg gggaagagtt ttggaagggg aggaaaatgg caagtgtatt    2066
tatattgtaa ccacatgcaa ataaaaagaa tgggaccta                           2105

<210>  17
<211>  435
<212>  PRT
<213>  Homo sapiens

<400>  17
Met Leu Leu Pro Trp Ala Thr Ser Ala Pro Gly Leu Ala Trp Gly Pro
1               5                   10                  15

Leu Val Leu Gly Leu Phe Gly Leu Leu Ala Ala Ser Gln Pro Gln Ala
            20                  25                  30

Val Pro Pro Tyr Ala Ser Glu Asn Gln Thr Cys Arg Asp Gln Glu Lys
        35                  40                  45

Glu Tyr Tyr Glu Pro Gln His Arg Ile Cys Cys Ser Arg Cys Pro Pro
    50                  55                  60

Gly Thr Tyr Val Ser Ala Lys Cys Ser Arg Ile Arg Asp Thr Val Cys
65                  70                  75                  80

Ala Thr Cys Ala Glu Asn Ser Tyr Asn Glu His Trp Asn Tyr Leu Thr
                85                  90                  95

Ile Cys Gln Leu Cys Arg Pro Cys Asp Pro Val Met Gly Leu Glu Glu
            100                 105                 110

Ile Ala Pro Cys Thr Ser Lys Arg Lys Thr Gln Cys Arg Cys Gln Pro
        115                 120                 125

Gly Met Phe Cys Ala Ala Trp Ala Leu Glu Cys Thr His Cys Glu Leu
    130                 135                 140

Leu Ser Asp Cys Pro Pro Gly Thr Glu Ala Glu Leu Lys Asp Glu Val
145                 150                 155                 160

Gly Lys Gly Asn Asn His Cys Val Pro Cys Lys Ala Gly His Phe Gln
                165                 170                 175

Asn Thr Ser Ser Pro Ser Ala Arg Cys Gln Pro His Thr Arg Cys Glu
            180                 185                 190

Asn Gln Gly Leu Val Glu Ala Ala Pro Gly Thr Ala Gln Ser Asp Thr
        195                 200                 205

Thr Cys Lys Asn Pro Leu Glu Pro Leu Pro Pro Glu Met Ser Gly Thr
    210                 215                 220

Met Leu Met Leu Ala Val Leu Leu Pro Leu Ala Phe Phe Leu Leu Leu
225                 230                 235                 240

Ala Thr Val Phe Ser Cys Ile Trp Lys Ser His Pro Ser Leu Cys Arg
                245                 250                 255

Lys Leu Gly Ser Leu Leu Lys Arg Arg Pro Gln Gly Glu Gly Pro Asn
            260                 265                 270

Pro Val Ala Gly Ser Trp Glu Pro Pro Lys Ala His Pro Tyr Phe Pro
        275                 280                 285

Asp Leu Val Gln Pro Leu Leu Pro Ile Ser Gly Asp Val Ser Pro Val
    290                 295                 300

Ser Thr Gly Leu Pro Ala Ala Pro Val Leu Glu Ala Gly Val Pro Gln
305                 310                 315                 320

Gln Gln Ser Pro Leu Asp Leu Thr Arg Glu Pro Gln Leu Glu Pro Gly
                325                 330                 335

Glu Gln Ser Gln Val Ala His Gly Thr Asn Gly Ile His Val Thr Gly
            340                 345                 350

Gly Ser Met Thr Ile Thr Gly Asn Ile Tyr Ile Tyr Asn Gly Pro Val
        355                 360                 365

Leu Gly Gly Pro Pro Gly Pro Gly Asp Leu Pro Ala Thr Pro Glu Pro
    370                 375                 380

Pro Tyr Pro Ile Pro Glu Glu Gly Asp Pro Gly Pro Pro Gly Leu Ser
385                 390                 395                 400

Thr Pro His Gln Glu Asp Gly Lys Ala Trp His Leu Ala Glu Thr Glu
                405                 410                 415

His Cys Gly Ala Thr Pro Ser Asn Arg Gly Pro Arg Asn Gln Phe Ile
            420                 425                 430

Thr His Asp
        435

<210> 18
<211> 903
<212> DNA
<213> Homo sapiens

<220>
<221> CDS
<222> (1)..(903)
<223>

<400> 18
atg agg gcg ctg gag ggg cca ggc ctg tcg ctg ctg tgc ctg gtg ttg 48
Met Arg Ala Leu Glu Gly Pro Gly Leu Ser Leu Leu Cys Leu Val Leu
1               5                   10                  15
gcg ctg cct gcc ctg ctg ccg gtg ccg gct gta cgc gga gtg gca gaa 96
Ala Leu Pro Ala Leu Leu Pro Val Pro Ala Val Arg Gly Val Ala Glu
            20                  25                  30
aca ccc acc tac ccc tgg cgg gac gca gag aca ggg gag cgg ctg gtg 144
Thr Pro Thr Tyr Pro Trp Arg Asp Ala Glu Thr Gly Glu Arg Leu Val
        35                  40                  45
tgc gcc cag tgc ccc cca ggc acc ttt gtg cag cgg ccg tgc cgc cga 192
Cys Ala Gln Cys Pro Pro Gly Thr Phe Val Gln Arg Pro Cys Arg Arg
    50                  55                  60
gac agc ccc acg acg tgt ggc ccg tgt cca ccg cgc cac tac acg cag 240
Asp Ser Pro Thr Thr Cys Gly Pro Cys Pro Pro Arg His Tyr Thr Gln
65                  70                  75                  80
ttc tgg aac tac ctg gag cgc tgc cgc tac tgc aac gtc ctc tgc ggg 288
Phe Trp Asn Tyr Leu Glu Arg Cys Arg Tyr Cys Asn Val Leu Cys Gly
                85                  90                  95
gag cgt gag gag gag gca cgg gct tgc cac gcc acc cac aac cgt gcc 336
Glu Arg Glu Glu Glu Ala Arg Ala Cys His Ala Thr His Asn Arg Ala
            100                 105                 110
tgc cgc tgc cgc acc ggc ttc ttc gcg cac gct ggt ttc tgc ttg gag 384
Cys Arg Cys Arg Thr Gly Phe Phe Ala His Ala Gly Phe Cys Leu Glu
        115                 120                 125
cac gca tcg tgt cca cct ggt gcc ggc gtg att gcc ccg ggc acc ccc 432
His Ala Ser Cys Pro Pro Gly Ala Gly Val Ile Ala Pro Gly Thr Pro
    130                 135                 140
agc cag aac acg cag tgc cag ccg tgc ccc cca ggc acc ttc tca gcc 480
Ser Gln Asn Thr Gln Cys Gln Pro Cys Pro Pro Gly Thr Phe Ser Ala
145                 150                 155                 160
agc agc tcc agc tca gag cag tgc cag ccc cac cgc aac tgc acg gcc 528
Ser Ser Ser Ser Ser Glu Gln Cys Gln Pro His Arg Asn Cys Thr Ala
                165                 170                 175
ctg ggc ctg gcc ctc aat gtg cca ggc tct tcc tcc cat gac acc ctg 576
Leu Gly Leu Ala Leu Asn Val Pro Gly Ser Ser Ser His Asp Thr Leu
            180                 185                 190
tgc acc agc tgc act ggc ttc ccc ctc agc acc agg gta cca gga gct 624
Cys Thr Ser Cys Thr Gly Phe Pro Leu Ser Thr Arg Val Pro Gly Ala
        195                 200                 205
gag gag tgt gag cgt gcc gtc atc gac ttt gtg gct ttc cag gac atc 672
Glu Glu Cys Glu Arg Ala Val Ile Asp Phe Val Ala Phe Gln Asp Ile
    210                 215                 220
tcc atc aag agg ctg cag cgg ctg ctg cag gcc ctc gag gcc ccg gag 720
Ser Ile Lys Arg Leu Gln Arg Leu Leu Gln Ala Leu Glu Ala Pro Glu
225                 230                 235                 240
ggc tgg ggt ccg aca cca agg gcg ggc cgc gcg gcc ttg cag ctg aag 768
Gly Trp Gly Pro Thr Pro Arg Ala Gly Arg Ala Ala Leu Gln Leu Lys
                245                 250                 255
ctg cgt cgg cgg ctc acg gag ctc ctg ggg gcg cag gac ggg gcg ctg 816
Leu Arg Arg Arg Leu Thr Glu Leu Leu Gly Ala Gln Asp Gly Ala Leu
            260                 265                 270

ctg gtg cgg ctg ctg cag gcg ctg cgc gtg gcc agg atg ccc ggg ctg 864
Leu Val Arg Leu Leu Gln Ala Leu Arg Val Ala Arg Met Pro Gly Leu
        275                 280                 285
gag cgg agc gtc cgt gag cgc ttc ctc cct gtg cac tga 903
Glu Arg Ser Val Arg Glu Arg Phe Leu Pro Val His
    290                 295                 300

<210> 19
<211> 300
<212> PRT
<213> Homo sapiens

<400> 19
Met Arg Ala Leu Glu Gly Pro Gly Leu Ser Leu Leu Cys Leu Val Leu
1               5                   10                  15

Ala Leu Pro Ala Leu Leu Pro Val Pro Ala Val Arg Gly Val Ala Glu
            20                  25                  30

Thr Pro Thr Tyr Pro Trp Arg Asp Ala Glu Thr Gly Glu Arg Leu Val
        35                  40                  45

Cys Ala Gln Cys Pro Pro Gly Thr Phe Val Gln Arg Pro Cys Arg Arg
    50                  55                  60

Asp Ser Pro Thr Thr Cys Gly Pro Cys Pro Pro Arg His Tyr Thr Gln
65                  70                  75                  80

Phe Trp Asn Tyr Leu Glu Arg Cys Arg Tyr Cys Asn Val Leu Cys Gly
                85                  90                  95

Glu Arg Glu Glu Glu Ala Arg Ala Cys His Ala Thr His Asn Arg Ala
            100                 105                 110

Cys Arg Cys Arg Thr Gly Phe Phe Ala His Ala Gly Phe Cys Leu Glu
        115                 120                 125

His Ala Ser Cys Pro Pro Gly Ala Gly Val Ile Ala Pro Gly Thr Pro
    130                 135                 140

Ser Gln Asn Thr Gln Cys Gln Pro Cys Pro Pro Gly Thr Phe Ser Ala
145                 150                 155                 160

Ser Ser Ser Ser Ser Glu Gln Cys Gln Pro His Arg Asn Cys Thr Ala
                165                 170                 175

Leu Gly Leu Ala Leu Asn Val Pro Gly Ser Ser Ser His Asp Thr Leu
            180                 185                 190

Cys Thr Ser Cys Thr Gly Phe Pro Leu Ser Thr Arg Val Pro Gly Ala
        195                 200                 205

Glu Glu Cys Glu Arg Ala Val Ile Asp Phe Val Ala Phe Gln Asp Ile
    210                 215                 220

Ser Ile Lys Arg Leu Gln Arg Leu Leu Gln Ala Leu Glu Ala Pro Glu
225                 230                 235                 240

Gly Trp Gly Pro Thr Pro Arg Ala Gly Arg Ala Ala Leu Gln Leu Lys
                245                 250                 255

Leu Arg Arg Arg Leu Thr Glu Leu Leu Gly Ala Gln Asp Gly Ala Leu
            260                 265                 270

Leu Val Arg Leu Leu Gln Ala Leu Arg Val Ala Arg Met Pro Gly Leu
        275                 280                 285

Glu Arg Ser Val Arg Glu Arg Phe Leu Pro Val His
    290                 295                 300

<210>  20
<211>  23
<212>  DNA
<213>  Artificial

<220>
<223>  Description of Artificial Sequence: Primer

<400>  20
atggagagtg tggtacagcc ttc                                              23

<210>  21
<211>  23
<212>  DNA
<213>  Artificial

<220>
<223>  Description of Artificial Sequence: Primer

<400>  21
gaccatgaaa gctccgaaat agg                                              23

<210>  22
<211>  22
<212>  DNA
<213>  Artificial

<220>
<223>  Description of Artificial Sequence: Primer

<400>  22
atggaacctc tcccaggatg gg                                               22

<210>  23
<211>  22
<212>  DNA
<213>  Artificial

<220>
<223>  Description of Artificial Sequence: Primer

<400>  23
tcagttggag gctgtctcct cc                                               22

## Description

### 1. FIELD OF THE INVENTION

The present invention relates to methods of treating or preventing immune disorders in a patient by combinatorial therapy methods. Such methods entail administering to a patient a combination of (1) a molecule that inhibits binding of LIGHT, a TNF-family ligand, to its receptor, the herpes virus entry mediator ("HVEM"); and (2) an immunosuppressive agent such as cyclosporine.

### 2. BACKGROUND OF THE INVENTION

By binding to structurally related but distinct receptors, tumor necrosis factor (TNF) cytokine family members play key roles in inflammation, immunity and homeostasis (Smith *et al*., 1994, Cell 76:959-62). An extensive literature exists on how antigenic stimulation of T cells in the absence of costimulation induces anergy, and addition of a second or costimulatory signal such as ligation of CD28 by B7-1 (CD80) or B7-2 (CD86) on dendritic cells promotes a primary T cell response. Various CD28-independent costimulatory pathways are also now recognized. In particular, accruing data suggest that TNF superfamily members can function as costimulatory molecules in T cell activation by delivering a second signal for T cell proliferation, cytokine production and Th1/Th2 differentiation (Locksley *et al*., 2001, Cell 104:487-501).

LIGHT is a cytokine that is homologous to lymphotoxins, exhibits inducible expression, and competes with herpes simplex virus (HSV) glycoprotein D for herpesvirus entry mediator (HVEM/TR2), a receptor expressed by T lymphocytes (Montgomery *et al*., 1996, Cell 87:427-36), is a recently identified member of the human (Mauri *et al*., 1998, Immunity 8:21-30) and mouse (Tamada *et al.,* 2000, Nat. Med. 6:283-9) TNF superfamily. LIGHT is a 29 kDa type II transmembrane protein produced by activated T cells (Mauri *et al*., 1998, Immunity 8:21-30), as well as monocytes and granulocytes (Zhai *et al*., 1998, J Clin Invest 102:1142-51), and immature dendritic cells (DC) (Tamada *et al*., 2000, J. Immunol. 164:4105-10). Apart from its receptor on T cells, HVEM, LIGHT binds to the lymphotoxin β receptor (LTβR) on stromal cells (Mauri *et al*., 1998, Immunity 8:21-30), and the DcR3/TR6 soluble protein (Yu *et al*., 1999, J Biol Chem 274:13733-6).

In vitro, LIGHT expression induces potent CD28-independent co-stimulatory activity, leading to NF-κB activation, production of IFN-γ and other cytokines, and T cell proliferation in response to allogeneic DC (Tamada *et al*., 2000, J. Immunol. 164:4105-10). In vivo studies show that LIGHT promotes cytolytic T cell responses to tumors (Tamada *et* *al*., 2000, Nat. Med. 6:283-9; Zhai *et al*., 1998, J Clin Invest 102:1142-51), as well as the development of graft versus host disease (GVHD) (Tamada *et al*., 2000, Nat. Med. 6:283-9), though no data are yet available concerning the role of the LIGHT/HVEM pathway in solid organ transplantation. The present invention arises from the data presented herein which shows that allograft rejection is repressed by inhibiting the HVEM-LIGHT interaction (by administering a molecule that inhibits the interaction or by disruption of the LIGHT gene) and administering an immunosuppressive agent to a graft recipient.

### 3. SUMMARY OF THE INVENTION

The present invention provides methods and compositions useful to treat or prevent immune system disorders such as allograft rejection. The present invention is based on the discovery that the administration of a combination of (a) a molecule that inhibits the HVEM-LIGHT interaction (referred to herein as an "HVEM-LIGHT inhibitor") and (b) an immunosuppressive agent results in potentiating administration of either agent alone, unlike what had been observed for administration of combinations of other immunosuppressive agents, such as the combination of anti-CD154 and cyclosporin A.

Accordingly, the present invention provides methods of treating or preventing an immune disorder in a patient, comprising administering to the patient (i) an HVEM-LIGHT inhibitor; and (ii) an immunosuppressive agent, in amounts effective for treating or preventing the immune disorder. The HVEM-LIGHT inhibitor and the immunosuppressive agent can be administered concurrently or successively. Preferably, the HVEM-LIGHT inhibitor and the immunosuppressive agent are administered in amounts that are synergistic.

The present invention provides pharmaceutical compositions comprising (1) an HVEM-LIGHT inhibitor; (2) an immunosuppressive agent; and (3) a pharmaceutically acceptable carrier, wherein the HVEM-LIGHT inhibitor and the immunosuppressive agent are present in amounts effective to prevent or treat the immune disorder.

The present invention further provides kits comprising in one or more containers (1) an HVEM-LIGHT inhibitor; (2) an immunosuppressive agent; (3) and instructions for the use of the HVEM-LIGHT inhibitor and the immunosuppressive agent in the prevention or treatment of immune disorder.

In the foregoing methods and compositions, preferred HVEM-LIGHT inhibitors are soluble HVEM or LIGHT proteins.

In other preferred embodiments, the HVEM-LIGHT inhibitor is an anti-HVEM antibody or an anti-LIGHT antibody.

In yet other preferred embodiments, the HVEM-LIGHT inhibitor is not one or more of the following: a herpesvirus gD protein, a lymphotoxin-α protein, a lymphotoxin-β receptor protein, or a TR6 receptor protein.

In a specific embodiment of the foregoing methods and compositions, for example where the immunosuppressive agent is a drug, the HVEM-LIGHT inhibitor can be conjugated to the immunosuppressive agent.

Immune disorders for which the present methods are useful for treating or preventing include autoimmune diseases, hypersensitivity, and allergic reactions. In a preferred embodiment, the immune disorder is allograft rejection.

The present invention further provides isolated mHVEM nucleic acid molecules, wherein the nucleic acid molecules are selected from the group consisting of: (a) a nucleic acid molecule having a nucleotide sequence which is at least 90% identical to the nucleotide sequence of SEQ ID NO:3 or a complement thereof as determined using the BLAST algorithm; (b) a nucleic acid molecule comprising at least 15 nucleotide residues and having a nucleotide sequence identical to at least 15 consecutive nucleotide residues of residues 1-424 of SEQ ID NO:3 or a complement thereof; (c) a nucleic acid molecule which encodes a polypeptide comprising the amino acid sequence of SEQ ID NO:4; (d) a nucleic acid molecule which encodes a fragment of a polypeptide comprising the amino acid sequence of any of SEQ ID NO:4, wherein the fragment comprises at least 12 consecutive amino acid residues of residues 1-80 of SEQ ID NO:4; and (e) a nucleic acid molecule which encodes a naturally occurring allelic variant of a polypeptide comprising the amino acid sequence of SEQ ID NO:4, wherein the nucleic acid molecule hybridizes under stringent conditions with a nucleic acid molecule consisting of the nucleotide sequence of any of residues 1-424 of SEQ ID NO:3 or a complement thereof.

The present invention further provides isolated mHVEM polypeptides, wherein the polypeptides are selected from the group consisting of: (a) a fragment of a polypeptide comprising the amino acid sequence of SEQ ID NO:4; wherein the fragment comprises at least 12 consecutive amino acid residues of residues 1-80 of SEQ ID NO:4; (b) a naturally occurring allelic variant of a polypeptide comprising the amino acid sequence of any of SEQ ID NO:4, wherein the polypeptide is encoded by a nucleic acid molecule which hybridizes with a nucleic acid molecule consisting of the nucleotide sequence of residues 1-424 of SEQ ID NO:3 or a complement thereof under stringent conditions; and (c) a polypeptide which is encoded by a nucleic acid molecule comprising a nucleotide sequence which is at least 90% identical to a nucleic acid consisting of residues 1-424 of SEQ ID NO:3 or a complement thereof as determined using the BLAST algorithm.

Host cells comprising the mHVEM nucleic acids of the invention are further provided. The present invention also encompasses method of making the mHVEM polypeptides of the invention comprising culturing host cells that contain the nucleic acids of the invention under conditions such that the mHVEM polypeptides are expressed. Yet further, the present invention further encompasses antibodies that bind to the mHVEM polypeptides of the invention but not to human HVEM polypeptides.

### 4. BRIEF DESCRIPTION OF THE FIGURE

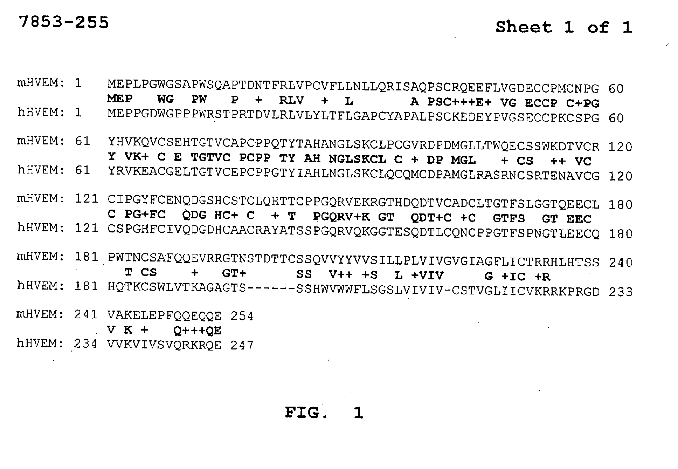

FIG. 1: Sequence alignment between the murine and human HVEM proteins. The sequences share 46% sequence identify and 57% sequence similarity. mHVEM = murine HVEM; hHVEM = human HVEM.

### 5. DETAILED DESCRIPTION OF THE INVENTION

The present invention provides methods and compositions useful to treat or prevent allograft rejection. The methods and compositions of the invention are based on the unexpected benefits observed when a combination of an (i) an HVEM-LIGHT inhibitor; and (ii) an immunosuppressive agent is administered to an allograft recipient. Thus, where prevention or treatment of allograft rejection is desired, the methods of the invention entail administering, concurrently or successively, to a patient, prior or following receipt of a transplant, a HVEM-LIGHT inhibitor and the immunosuppressive agent. Such methods are described in more detail in Section 5.1, *infra*.

The present invention provides pharmaceutical compositions and kits that are useful for practicing the methods of the invention. Such pharmaceutical compositions and kits are described in Sections 5.5 and 5.6 below, respectively.

### 5.1 METHODS OF THE INVENTION

Described below are combinatorial methods and related compositions for treating or preventing immune disorders such as allograft rejection. The methods of the invention involve the administration of at least two agents to a patient, the first of which disrupts the interaction between the HVEM receptor and its ligand, LIGHT, and the second of which is an immunosuppressive agent. The agent which disrupts the interaction between HVEM and LIGHT is referred to herein as an HVEM-LIGHT inhibitor. An HVEM-LIGHT inhibitor preferably disrupts the interaction between the HVEM receptor and its ligand LIGHT by at least 20%, more preferably by at least 30%, more preferably by at least 40%, yet more preferably by at least 50%. In certain embodiments, an HVEM-LIGHT inhibitor disrupts the interaction between HVEM and LIGHT by up to 60%, 70%, 80%, or 90%. As used herein, percentage inhibition of the HVEM-LIGHT interaction is measured according to an embodiment of the heterogenous assay described in Section 5.5, *infra*. Briefly, a protein (such as a fusion protein) comprising a LIGHT-binding portion of HVEM (or an HVEM-binding portion of LIGHT) is immobilized on a solid surface, and contacted with a protein comprising an HVEM-binding portion of LIGHT (or a LIGHT-binding portion of HVEM) in the presence and absence of the test compound. After the reaction is complete, unreacted components are removed (*e.g*., by washing) and any complexes formed will remain immobilized on the solid surface. A radioactively labeled antibody that binds to the HVEM-binding portion of LIGHT (or to the LIGHT-binding portion of HVEM), but not to the test compound, can be added to the system and allowed to bind to the complexed components. The interaction between HVEM and LIGHT can be detected by measuring the amount of radioactivity that remains associated with the HVEM-LIGHT complex. A successful inhibition of the interaction by the test compound will result in a decrease in measured radioactivity. The % inhibition of the HVEM -LIGHT interaction is the percentage difference in bound radioactivity in the present and absence of test compound; for example, if the amount of bound radioactivity in the presence of the test compound is 70% of bound radioactivity in the absence of the test compound, the test compound is said to inhibit the HVEM-LIGHT interaction by 30%.

The HVEM-LIGHT inhibitor can be a competitive or non-competitive inhibitor of the HVEM-LIGHT interaction. As described in Section 5.2, *infra*, the HVEM-LIGHT inhibitor can be a protein. In one embodiment, the HVEM-LIGHT inhibitor is a membrane-bound form of LIGHT or HVEM, for example LIGHT or HVEM recombinantly expressed on a cell. For example, an HVEM-expressing cell that does not contain the machinery for mediating the LIGHT signal can be used to inhibit the endogenous HVEM-LIGHT interaction. In more preferred embodiments, the HVEM-LIGHT inhibitor is a soluble protein. In one embodiment, the HVEM-LIGHT inhibitor is a soluble form of HVEM or a soluble form of another receptor to which LIGHT binds. In another embodiment, the HVEM-LIGHT inhibitor is a soluble LIGHT protein or another ligand which binds to HVEM. In yet other embodiments, the HVEM-LIGHT inhibitor is an anti-HVEM or anti-LIGHT antibody. Alternatively, the HVEM-LIGHT inhibitor can be small organic or inorganic molecule of preferably less than 500 daltons in size.

The combinatorial therapy methods of the present invention often result in a greater than additive effect, providing therapeutic benefits where neither the HVEM-LIGHT inhibitor or immunosuppressive agent alone is effective. The outcome of the present therapeutic and prophylactic methods is to at least produce in a patient a healthful benefit, which includes but is not limited to: prolonging the lifespan of a patient, prolonging the onset of symptoms of the disorder, and/or alleviating a symptom of the disorder after onset of a symptom of the disorder. For example, in the context of allograft rejection, the present therapeutic and prophylactic methods can result in prolonging the lifespan of an allograft recipient, prolonging the duration of allograft tolerance prior to rejection, and/or alleviating a symptom associated with allograft rejection.

As used herein, the term "prevention" refers to administration of one or both components of the combinatorial regimens of the invention to the patient before the onset of symptoms or molecular indications of the immune disorder of interest. In contrast, the term "treatment" refers to administration of one or both components of the combinatorial regimens of the present invention to the patient after the onset of symptoms or molecular indications of the disorder. With respect to allograft rejection or graft versus host disease, in a preferred embodiment of the invention, the prophylactic methods of the invention are initiated prior to transplantation of the allograft.

The patients on whom the combinatorial therapy methods of the invention are practiced include, but are not limited to, animals such as cows, pigs, horses, chickens, cats, dogs, *etc.*, and are preferably mammals, and most preferably human.

In the present methods, the HVEM-LIGHT inhibitor and the immunosuppressive agent can be administered concurrently or successively. As used herein, the HVEM-LIGHT inhibitor and the immunosuppressive agent are said to be administered concurrently if they are administered to the patient on the same day, for example, simultaneously, or 1, 2, 3, 4, 5, 6, 7 or 8 hours apart. In contrast, the HVEM-LIGHT inhibitor and the immunosuppressive agent are said to be administered successively if they are administered to the patient on the different days, for example, the HVEM-LIGHT inhibitor and the immunosuppressive agent can be administered at a 1-day, 2-day or 3-day intervals. In the methods of the present invention, administration of the HVEM-LIGHT inhibitor can precede or follow administration of the immunosuppressive agent.

The therapeutic and prophylactic regimens of the present invention can include both concurrent and successive administration. As a non-limiting example, the HVEM-LIGHT inhibitor and immunosuppressive agent can be administered concurrently for a period of time, followed by a second period of time in which the administration of the HVEM-LIGHT inhibitor and the immunosuppressive agent is alternated.

The therapeutic regimens of the present invention can be practiced as long as the treatment or prevention of an immune disorder is required or desired.

Because of the synergistic effects of administering a HVEM-LIGHT inhibitor and an immunosuppressive agent, such agents can be administered in amounts that, if one or both of the agents is administered alone, is/are not effective for treating an immune disorder of interest.

### 5.1.1 ALLOGRAFT REJECTION AND GRAFT VERSUS HOST DISEASE

In preferred embodiments of the present invention, the methods and compositions of the present invention are used to treat or prevent allograft rejection or graft versus host disease.

The methods and compositions of the invention can be used in the prevention or treatment of any type of allograft rejection or graft versus host disease for any type of graft, including a xenograft. The allograft can be an organ transplant, such as, but not limited to, a heart, kidney, liver, lung or pancreas. Alternatively, the allograft can be a tissue transplant, such as, but not limited to, heart valve, endothelial, cornea, eye lens or bone marrow tissue transplant. In yet other embodiments, the allograft can be a skin graft.

In certain embodiments, the present methods can be practiced for a day, three days, a week, two weeks or a month prior to a transplantation. In other embodiments, the present methods can be practiced for a week, two weeks, three weeks, one month, two months, three months or six months following a transplantation. In a preferred embodiment, the present methods are practiced both before and after a transplantation is carried out.

### 5.1.2 DISORDERS OF THE INVENTION

The methods and compositions of the present invention are useful for treating or preventing a variety of immune disorders, including but not limited to autoimmune diseases, hypersensitivity, and allergic reactions. Such disorders are referred to herein as disorders of the invention.

Exemplary disorders of the invention include allograft rejection, delayed-type hypersensitivity, graft versus host disease, drug allergies, atrophic gastrititis, thyroiditis, allergic encephalomyelitis, autoimmune hemolytic anemia, sympathetic ophthalmia, systemic lupus erythematosus ("SLE"), rheumatoid arthritis, multiple sclerosis, asthma, inflammatory bowel disease ("IBD"), and myasthenia gravis.

### 5.2 HVEM-LIGHT INHIBITORS

As mentioned in Section 5.1, *supra,* many types of molecules can be used as HVEM-LIGHT inhibitors. Such molecules include polypeptides, peptides, antibodies, and small molecules. Below is a description of exemplary HVEM-LIGHT inhibitors. In certain specific embodiments of the invention, the HVEM-LIGHT inhibitor employed in the present methods and compositions is not one or more of the following: a herpesvirus gD protein, a lymphotoxin-α protein, a lymphotoxin-β receptor protein, or a TR6 receptor protein.

### 5.2.1 DOMINANT NEGATIVE FORMS OF LIGHT AND HVEM-BINDING POLYPEPTIDES

The present invention encompasses the use of dominant negative forms of polypeptides that bind to the HVEM or LIGHT proteins in the methods and compositions of the present invention. Such dominant negative proteins include full length HVEM proteins (for example, expressed by a cell that is administered to a patient) or peptide fragments thereof which bind to the LIGHT ligand, as well as full length LIGHT proteins (for example, expressed by a cell that is administered to a patient) or peptide fragments thereof which bind to the HVEM receptor.

Also included are dominant negative forms of HVEM ligands other than LIGHT (*e.g*., herpesvirus gD protein (Genbank accession no. L09242; SEQ ID NO:13), lymphotoxin-α Genbank accession no. AY070490; SEQ ID NO: 15), as well as dominant negative forms of receptors to which LIGHT binds other than HVEM (*e.g*., the lymphotoxin-β receptor (Genbank accession no. XM_033305 SEQ ID NO:17), the TR6 receptor (Genbank accession no. AF134240; SEQ ID NO:19; see also Yu *et al*., 1999, J. Biol. Chem. 274:13733-36)).

The amino acid sequences depicted in SEQ ID NO:2 (see WO 00/14230, where HVEM is referred to as TANGO-69-receptor) and SEQ ID NO:4 represent full length human and murine HVEM proteins, respectively. The amino acid sequences depicted in SEQ ID NO:6 (see Genbank accession no. AF036581) and SEQ ID NO:8 (see WO 99/11662, where LIGHT is referred to as TANGO-69) represent full length human and murine LIGHT proteins, respectively.

Preferred in the present methods ane compositions are soluble LIGHT and HVEM polypeptides. Such polypeptides generally lack a transmembrane domain and an intracellular domain.

After HVEM is processed into a mature protein, the extracellular domain of human HVEM consists of amino acids 39-201 of the full protein (SEQ ID NO:2) (Whitbeck *et al*., 2001, J. Virol. 75:171-80). The use of the entire HVEM extracellular domain, or a LIGHT binding portion thereof, is contemplated in the present methods an compositions. WO 00/14230 teaches that there are three known soluble forms of the normally membrane-bound HVEM, referred to sHVEM1 (SEQ ID NO:9), sHVEM2 (SEQ ID NO:10), and sHVEM3 (SEQ ID NO:11). The use of these soluble forms, or a LIGHT-binding portion thereof, is also desirable in the present methods.

Fragments of HVEM or LIGHT that are useful in the methods and compositions present invention may contain deletions, additions or substitutions of amino acid residues within the amino acid sequence encoded by an HVEM or LIGHT gene. Preferably mutations result in a silent change, thus producing a functionally equivalent HVEM or LIGHT gene product.

An HVEM or LIGHT polypeptide sequence preferably comprises an amino acid sequence that exhibits at least about 65% sequence similarity to human HVEM or LIGHT, more preferably exhibits at least 70% sequence similarity to human HVEM or LIGHT, yet more preferably exhibits at least about 75% sequence similarity human HVEM or LIGHT. In other embodiments, the HVEM or LIGHT polypeptide sequence preferably comprises an amino acid sequence that exhibits at least 85% sequence similarity to human HVEM or LIGHT, yet more preferably exhibits at least 90% sequence similarity to human HVEM or LIGHT, and most preferably exhibits at least about 95% sequence similarity to human HVEM or LIGHT. In one embodiment, such a polypeptide sequence comprises all or a portion of the murine HVEM or LIGHT sequence, respectively.

In other embodiment, the HVEM or LIGHT polypeptide sequence preferably comprises an amino acid sequence that exhibits at least about 65% sequence identity to marine HVEM or LIGHT, more preferably exhibits at least 70% sequence identity to murine HVEM or LIGHT, yet more preferably exhibits at least about 75% sequence identity to murine HVEM or LIGHT. In other embodiments, the HVEM or LIGHT polypeptide sequence preferably comprises an amino acid sequence that exhibits at least 85% sequence identity to murine HVEM or LIGHT, yet more preferably exhibits at least 90% sequence identity to murine HVEM or LIGHT, and most preferably exhibits at least about 95% sequence identity to murine HVEM or LIGHT. In one embodiment, such a polypeptide sequence comprises a portion of murine HVEM that binds to the human LIGHT extracellular domain, or a portion of murine LIGHT that binds to the human HVEM extracellular domain, respectively.

Additional polypeptides suitable in the present methods are those encoded by the nucleic acids described in Section 5.4.1 below.

The determination of percent identity between two sequences can be accomplished using a mathematical algorithm. A preferred, non-limiting example of a mathematical algorithm utilized for the comparison of two sequences is the algorithm of Karlin and Altschul (1990) *Proc Natl Acad Sci*. 87:2264-2268, modified as in Karlin and Altschul (1993) *Proc Natl Acad Sci.* 90:5873-5877. Such an algorithm is incorporated into the NBLAST and XBLAST programs of Altschul *et al*. (1990) *J*. *Mol. Biol.* 215:403-410. BLAST nucleotide searches can be performed with the NBLAST program, score = 100, wordlength = 12 to obtain nucleotide sequences homologous to a nucleic acid molecules of the invention. BLAST protein searches can be performed with the XBLAST program, score = 50, wordlength = 3 to obtain amino acid sequences homologous to a protein molecules of the invention. To obtain gapped alignments for comparison purposes, Gapped BLAST can be utilized as described in Altschul et al. (1997) *Nucleic Acids Res.* 25:3389-3402. Alternatively, PSI-Blast can be used to perform an iterated search which detects distant relationships between molecules (*Id*.). When utilizing BLAST, Gapped BLAST, and PSI-Blast programs, the default parameters of the respective programs (*e.g*., XBLAST and NBLAST) can be used.

Another preferred, non-limiting example of a mathematical algorithm utilized for the comparison of sequences is the algorithm of Myers and Miller, (1988) CABIOS 4:11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Additional algorithms for sequence analysis are known in the art and include ADVANCE and ADAM as described in Torellis and Robotti (1994) *Comput. Appl. Biosci.,* 10:3-5; and FASTA described in Pearson and Lipman (1988) 85:2444-8. Within FASTA, ktup is a control option that sets the sensitivity and speed of the search. If ktup=2, similar regions in the two sequences being compared are found by looking at pairs of aligned residues; if ktup=1, single aligned amino acids are examined. ktup can be set to 2 or 1 for protein sequences, or from 1 to 6 for DNA sequences. The default if ktup is not specified is 2 for proteins and 6 for DNA. For a further description of FASTA parameters, see http://bioweb.pasteur.fr/docs/man/man/fasta. 1.htm1#sect2.

The percent identity between two sequences can be determined using techniques similar to those described above, with or without allowing gaps. In calculating percent identity, only exact matches are counted. However, conservative substitutions should be considered in evaluating sequences that have a low percent identity with the HVEM or LIGHT sequences disclosed herein.

In a specific embodiment, polypeptides comprising at least 10, 20, 30, 40, 50, 75, 100, or 200 amino acids of SEQ ID NO:2 or 4 that bind to LIGHT, or polypeptides comprising at least 10, 20, 30, 40, 50, 75, 100, or 200 amino acids of SEQ ID NO:6 or 8 that bind to HVEM, are used in the present invention. In a preferred embodiment, such a polypeptide comprises all or a portion of the extracellular domain of SEQ ID NO:2, 4, 6, or 8.

### 5.2.1.1 FUSION PROTEINS

Also useful in the present methods and compositions also are fusion proteins comprising a portion of an HVEM-binding ligand or a LIGHT-binding receptor polypeptide sequence which binds to HVEM or LIGHT, respectively, operatively associated to a heterologous component, e.g., a heterologous peptide. Heterologous components can include, but are not limited to sequences which facilitate isolation and purification of the fusion protein. Heterologous components can also include sequences which confer stability to the LIGHT- or HVEM-binding polypeptides. Such fusion partners are well known to those of skill in the art.

The present invention encompasses the use of fusion proteins comprising an HVEM (*e.g*., SEQ ID NO:2 or SEQ ID NO:4) or LIGHT polypeptide (SEQ ID NO:6 and SEQ ID NO:8) and a heterologous polypeptide (*i.e*., an unrelated polypeptide or fragment thereof, preferably at least 10, at least 20, at least 30, at least 40, at least 50, at least 60, at least 70, at least 80, at least 90 or at least 100 amino acids of the polypeptide). The present invention encompasses the use of fusion proteins comprising an herpesvirus gD protein (SEQ ID NO:13), lymphotoxin-α (SEQ ID NO:15), the lymphotoxin-β receptor (SEQ ID NO: 17), or the TR6 receptor (SEQ ID NO:19) and a heterologous polypeptide *(i.e.,* an unrelated polypeptide or fragment thereof, preferably at least 10, at least 20, at least 30, at least 40, at least 50, at least 60, at least 70, at least 80, at least 90 or at least 100 amino acids of the polypeptide). The fusion can be direct, but may occur through linker sequences. The heterologous polypeptide may be fused to the N-terminus or C-terminus of an LIGHT- or HVEM-binding polypeptide.

A fusion protein can comprise an LIGHT- or HVEM-binding polypeptide fused to a heterologous signal sequence at its N-terminus. Various signal sequences are commercially available. Eukaryotic heterologous signal sequences include, but art not limited to, the secretory sequences of melittin and human placental alkaline phosphatase (Stratagene; La Jolla, California). Prokaryotic heterologous signal sequences useful in the methods of the invention include, but are not limited to, the phoA secretory signal (Sambrook *et al*., eds., *Molecular Cloning: A Laboratory Manual, 2nd ed., Cold Spring Harbor Laboratory*, Cold Spring Harbor Laboratory Press, Cold Spring Harbor, NY, 1989) and the protein A secretory signal (Pharmacia Biotech; Piscataway, New Jersey).

The LIGHT- or HVEM-binding protein or fragment thereof can be fused to tag sequences, *e.g*., a hexa-histidine peptide, such as the tag provided in a pQE vector (QIAGEN, Inc., Chatsworth, CA, 91311), among others, many of which are commercially available for use in the methods of the invention. As described in Gentz *et al*., 1989, *Proc. Natl. Acad. Sci. USA*, 86:821-824, for instance, hexa-histidine provides for convenient purification of the fusion protein. Other examples of peptide tags are the hemagglutinin "HA" tag, which corresponds to an epitope derived from the influenza hemagglutinin protein (Wilson *et al*., 1984, *Cell,* 37:767) and the "flag" tag (Knappik *et al*., 1994, *Biotechniques*, 17(4):754-761). These tags are especially useful for purification of recombinantly produced polypeptides of the invention.

Any fusion protein may be readily purified by utilizing an antibody specific or selective for the fusion protein being expressed. For example, a system described by Janknecht *et al*. allows for the ready purification of non-denatured fusion proteins expressed in human cell lines (Janknecht *et al*., 1991, *Proc. Natl. Acad. Sci. USA* 88:8972). In this system, the gene of interest is subcloned into a vaccinia recombination plasmid such that the open reading frame of the gene is translationally fused to an amino-terminal tag consisting of six histidine residues. Extracts from cells infected with recombinant vaccinia virus are loaded onto Ni²⁺·nitriloacetic acid-agarose columns and histidine-tagged proteins are selectively eluted with imidazole-containing buffers.

An affinity label can also be fused at its amino terminal to the carboxyl terminal of the LIGHT- or HVEM-binding protein or fragment thereof for use in the methods of the invention. The precise site at which the fusion is made in the carboxyl terminal is not critical. The optimal site can be determined by routine experimentation. An affinity label can also be fused at its carboxyl terminal to the amino terminal of the LIGHT-or HVEM-binding polypeptide for use in the methods of the invention.

A variety of affinity labels known in the art may be used, such as, but not limited to, the immunoglobulin constant regions (see also Petty, 1996, Metal-chelate affinity chromatography, in Current Protocols in Molecular Biology, Vol. 2, Ed. Ausubel *et al*., Greene Publish. Assoc. & Wiley Interscience), glutathione S-transferase (GST; Smith, 1993, *Methods Mol. Cell Bio.* 4:220-229), the *E. coli* maltose binding protein (Guan *et al*., 1987, *Gene* 67:21-30), and various cellulose binding domains (U.S. Patent Nos. 5,496,934; 5,202,247; 5,137,819; Tomme *et al*., 1994, *Protein Eng.* 7:117-123), etc. Other affinity labels are recognized by specific binding partners and thus facilitate isolation by affinity binding to the binding partner which can be immobilized onto a solid support. Some affinity labels may afford the LIGHT- or HVEM-binding polypeptide novel structural properties, such as the ability to form multimers. These affinity labels are usually derived from proteins that normally exist as homopolymers. Affinity labels such as the extracellular domains of CD8 (Shiue *et al*., 1988, *J. Exp. Med.* 168:1993-2005), or CD28 (Lee *et al*., 1990, *J. Immunol.* 145:344-352), or fragments of the immunoglobulin molecule containing sites for interchain disulfide bonds, could lead to the formation of multimers.

As will be appreciated by those skilled in the art, many methods can be used to obtain the coding region of the above-mentioned affinity labels, including but not limited to, DNA cloning, DNA amplification, and synthetic methods. Some of the affinity labels and reagents for their detection and isolation are available commercially.

A preferred affinity label is a non-variable portion of the immunoglobulin molecule. Typically, such portions comprise at least a functionally operative CH2 and CH3 domain of the constant region of an immunoglobulin heavy chain. Fusions are also made using the carboxyl terminus of the Fc portion of a constant domain, or a region immediately amino-terminal to the CH1 of the heavy or light chain. Suitable immunoglobulin-based affinity label may be obtained from IgG-1, -2, -3, or -4 subtypes, IgA, IgE, IgD, or IgM, but preferably IgG1. Preferably, a human immunoglobulin is used when the LIGHT- or HVEM-binding polypeptide is intended for *in vivo* use for humans. Many DNA encoding immunoglobulin light or heavy chain constant regions are known or readily available from cDNA libraries. See, for example, Adams *et al., Biochemistry,* 1980, 19:2711-2719; Gough *et al*., 1980, *Biochemistry,* 19:2702-2710; Dolby *et al*., 1980, *Proc. Natl. Acad. Sci. U.S.A*., 77:6027-6031; Rice *et al*., 1982, *Proc. Natl. Acad. Sci. U.S.A*., 79:7862-7865; Falkner *et al*., 1982, *Nature,* 298:286-288; and Morrison *et al*., 1984, *Ann. Rev. Immunol,* 2:239-256. Because many immunological reagents and labeling systems are available for the detection of immunoglobulins, the LIGHT- or HVEM-binding polypeptide-Ig fusion protein can readily be detected and quantified by a variety of immunological techniques known in the art, such as the use of enzyme-linked immunosorbent assay (ELISA), immunoprecipitation, fluorescence activated cell sorting (FACS), *etc.* Similarly, if the affinity label is an epitope with readily available antibodies, such reagents can be used with the techniques mentioned above to detect, quantitate, and isolate the LIGHT- or HVEM-binding polypeptide containing the affinity label. In many instances, there is no need to develop specific or selective antibodies to the LIGHT- or HVEM-binding polypeptide for the purposes of purification.

A fusion protein can comprise an LIGHT- or HVEM-binding polypeptide fused to the Fc domain of an immunoglobulin molecule or a fragment thereof for use in the methods of the invention. A fusion protein can also comprise an LIGHT- or HVEM-binding polypeptide fused to the CH2 and/or CH3 region of the Fc domain of an immunoglobulin molecule. Furthermore, a fusion protein can comprise an LIGHT- or HVEM-binding polypeptide fused to the CH2, CH3, and hinge regions of the Fc domain of an immunoglobulin molecule (see Bowen *et al*., 1996, *J. Immunol.* 156:442-49). This hinge region contains three cysteine residues which are normally involved in disulfide bonding with other cysteines in the Ig molecule. Since none of the cysteines are required for the peptide to function as a tag, one or more of these cysteine residues may optionally be substituted by another amino acid residue, such as for example, serine.

Various leader sequences known in the art can be used for the efficient secretion of the LIGHT- or HVEM-binding polypeptide from bacterial and mammalian cells (von Heijne, 1985, *J. Mol. Biol.* 184:99-105). Leader peptides are selected based on the intended host cell, and may include bacterial, yeast, viral, animal, and mammalian sequences. For example, the herpes virus glycoprotein D leader peptide is suitable for use in a variety of mammalian cells. A preferred leader peptide for use in mammalian cells can be obtained from the V-J2-C region of the mouse immunoglobulin kappa chain (Bernard *et al*., 1981, *Proc. Natl. Acad. Sci.* 78:5812-5816). Preferred leader sequences for targeting HVEM or LIGHT polypeptide expression in bacterial cells include, but are not limited to, the leader sequences of the *E.coli* proteins OmpA (Hobom *et al*., 1995, *Dev. Biol. Stand.* 84:255-262), Pho A (Oka *et al*., 1985, *Proc. Natl. Acad. Sci* 82:7212-16), OmpT (Johnson *et al*., 1996, *Protein Expression* 7:104-113), LamB and OmpF (Hoffman & Wright, 1985, *Proc. Natl. Acad. Sci. USA* 82:5107-5111), β-lactamase (Kadonaga *et al*., 1984, *J. Biol. Chem.* 259:2149-54), enterotoxins (Morioka-Fujimoto *et al*., 1991, *J. Biol. Chem.* 266:1728-32), and the *Staphylococcus aureus* protein A (Abrahmsen *et al*., 1986, *Nucleic Acids Res.* 14:7487-7500), and the *B. subtilis* endoglucanase (Lo *et al*., *Appl. Environ. Microbiol.* 54:2287-2292), as well as artificial and synthetic signal sequences (MacIntyre *et al*., 1990, *Mol. Gen*. *Genet.* 221:466-74; Kaiser *et al*., 1987, *Science,* 235:312-317).

Fusion proteins can be produced by standard recombinant DNA techniques or by protein synthetic techniques, *e.g*., by use of a peptide synthesizer. For example, a nucleic acid molecule encoding a fusion protein can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (*see, e.g., Current Protocols in Molecular Biology,* Ausubel *et al*., eds., John Wiley & Sons, 1992).

The nucleotide sequence coding for a fusion protein can be inserted into an appropriate expression vector, *i.e*., a vector which contains the necessary elements for the transcription and translation of the inserted protein-coding sequence. The expression of a fusion protein may be regulated by a constitutive, inducible or tissue-specific or -selective promoter. It will be understood by the skilled artisan that fusion proteins, which can facilitate solubility and/or expression, and can increase the *in vivo* half-life of the LIGHT- or HVEM-binding polypeptide and thus are useful in the methods of the invention. The LIGHT- or HVEM-binding polypeptides or peptide fragments thereof, or fusion proteins can be used in any assay that detects or measures LIGHT- or HVEM-binding polypeptides or in the calibration and standardization of such assay.

The methods of invention encompass the use of LIGHT- or HVEM-binding polypeptides or peptide fragments thereof, which may be produced by recombinant DNA technology using techniques well known in the art. Thus, methods for preparing the LIGHT- or HVEM-binding polypeptides and peptides of the invention by expressing nucleic acid containing LIGHT- or HVEM-binding gene sequences are described herein. Methods which are well known to those skilled in the art can be used to construct expression vectors containing, *e.g*., HVEM polypeptide coding sequences (including but not limited to nucleic acids encoding all or a LIGHT-binding portion of SEQ ID NO:9, SEQ ID NO:10, or SEQ ID NO:11) or LIGHT polypeptide coding sequences and appropriate transcriptional and translational control signals. These methods include, for example, *in vitro* recombinant DNA techniques, synthetic techniques, and *in vivo* genetic recombination. See, for example, the techniques described in Sambrook *et al*., 1989, *supra,* and Ausube1 *et al*., 1989, *supra.* Alternatively, RNA capable of encoding LIGHT- or HVEM-binding polypeptide sequences may be chemically synthesized using, for example, synthesizers (*see e.g*., the techniques described in *Oligonucleotide Synthesis,* 1984, Gait, M.J. ed., IRL Press, Oxford).

### 5.2.1.2 EXPRESSION SYSTEMS

A variety of host-expression vector systems may be utilized to express the LIGHT- or HVEM-binding polypeptide coding sequences for use in the methods of the invention. Such host-expression systems represent vehicles by which the coding sequences of interest may be produced and subsequently purified, but also represent cells which may, when transformed or transfected with the appropriate nucleotide coding sequences, exhibit the LIGHT- or HVEM-binding polypeptide of the invention *in situ*. These include but are not limited to microorganisms such as bacteria (*e.g., E. coli, B. subtilis*) transformed with recombinant bacteriophage DNA, plasmid DNA or cosmid DNA expression vectors containing LIGHT- or HVEM-binding polypeptide coding sequences; yeast (*e.g*., *Saccharomyces, Pichia*) transformed with recombinant yeast expression vectors containing the LIGHT- or HVEM-binding polypeptide coding sequences; insect cell systems infected with recombinant virus expression vectors (*e.g*., baculovirus) containing the LIGHT- or HVEM-binding polypeptide coding sequences; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing LIGHT- or HVEM-binding polypeptide coding sequences; or mammalian cell systems (*e.g*., COS, CHO, BHK, 293, 3T3) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter).

In bacterial systems, a number of expression vectors may be advantageously selected depending upon the use intended for the LIGHT- or HVEM-binding polypeptide being expressed. For example, when a large quantity of such a protein is to be produced, for the generation of pharmaceutical compositions of LIGHT- or HVEM-binding protein or for raising antibodies to HVEM or LIGHT protein, vectors which direct the expression of high levels of fusion protein products that are readily purified may be desirable. Such vectors include, but are not limited, to the *E. coli* expression vector pUR278 (Ruther *et al*., 1983, *EMBO J*. 2:1791), in which the HVEM or LIGHT polypeptide coding sequence may be ligated individually into the vector in frame with the *lac Z* coding region so that a fusion protein is produced; pIN vectors (Inouye & Inouye, 1985, *Nucleic Acids Res.* 13:3101; Van Heeke & Schuster, 1989, *J. Biol. Chem.* 264:5503); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include, *e.g*.,thrombin or factor Xa protease cleavage sites so that the cloned target polypeptide can be released from the GST moiety.

In an insect system, *Autographa californica* nuclear polyhedrosis virus (AcNPV) is used as a vector to express foreign genes. The virus grows in *Spodoptera frugiperda* cells. The LIGHT- or HVEM-binding polypeptide coding sequence may be cloned individually into non-essential regions (for example the polyhedrin gene) of the virus and placed under control of an AcNPV promoter (for example the polyhedrin promoter). Successful insertion of LIGHT- or HVEM-binding polypeptide coding sequence will result in inactivation of the polyhedrin gene and production of non-occluded recombinant virus (*i.e*., virus lacking the proteinaceous coat coded for by the polyhedrin gene). These recombinant viruses are then used to infect Spodoptera frugiperda cells in which the inserted gene is expressed (*e.g*., see Smith *et al*., 1983, *J. Virol.* 46:584; Smith, U.S. Patent No. 4,215,051).

In mammalian host cells, a number of viral-based expression systems may be utilized. In cases where an adenovirus is used as an expression vector, the LIGHT- or HVEM-binding polypeptide coding sequence of interest may be ligated to an adenovirus transcription/translation control complex, *e.g*., the late promoter and tripartite leader sequence. This chimeric gene may then be inserted in the adenovirus genome by *in vitro* or *in vivo* recombination. Insertion in a non-essential region of the viral genome (*e.g*., region E1 or E3) will result in a recombinant virus that is viable and capable of expressing HVEM or LIGHT polypeptide in infected hosts. (*See, e.g*., Logan & Shenk, 1984, *Proc. Natl. Acad. Sci. USA* 81:3655). Specific initiation signals may also be required for efficient translation of inserted LIGHT- or HVEM-binding polypeptide coding sequences. These signals include the ATG initiation codon and adjacent sequences. In cases where an entire LIGHT-or HVEM-binding polypeptide gene, including its own initiation codon and adjacent sequences, is inserted into the appropriate expression vector, no additional translational control signals may be needed. However, in cases where only a portion of the LIGHT- or HVEM-binding polypeptide coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (*See* Bittner *et al*., 1987, *Methods in Enzymol.* 153:516).

In addition, a host cell strain may be chosen which modulates the expression of the inserted sequences, or modifies and processes the polypeptide in the specific fashion desired. Such modifications (*e.g*., glycosylation) and processing (*e.g*., cleavage) of protein products may be important for the function of the protein. Different host cells have characteristic and specific mechanisms for the post-translational processing and modification of proteins and polypeptides. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the polypeptide may be used. Such mammalian host cells include but are not limited to CHO, VERO, BHK, HeLa, COS, MDCK, 293, 3T3, WI38.

For long-term, high-yield production of recombinant proteins, stable expression is preferred. For example, cell lines which stably express the LIGHT- or HVEM-binding polypeptide may be engineered. Rather than using expression vectors which contain viral origins of replication, host cells can be transformed with DNA controlled by appropriate expression control elements (*e.g.*, promoter, enhancer, sequences, transcription terminators, polyadenylation sites, etc.), and a selectable marker. Following the introduction of the foreign DNA, engineered cells maybe allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the LIGHT- or HVEM-binding polypeptide. Such engineered cell lines may be particularly useful in screening and evaluation of compounds that affect the endogenous activity of the HVEM or LIGHT polypeptide.

A number of selection systems may be used, including but not limited to the herpes simplex virus thymidine kinase (Wigler *et al*., 1977, *Cell* 11:223), hypoxanthine-guanine phosphoribosyltransferase (Szybalska & Szybalski, 1962, *Proc. Natl. Acad. Sci. USA* 48:2026), and adenine phosphoribosyltransferase (Lowy *et al*., 1980, *Cell* 22:817) genes can be employed in tk⁻, hgprt- or aprt⁻ cells, respectively. Also, antimetabolite resistance can be used as the basis of selection for the following genes: dhfr, which confers resistance to methotrexate (Wigler *et al*., 1980, *Proc Natl. Acad. Sci. USA* 77:3567; O'Hare *et al*., 1981, *Proc. Natl. Acad. Sci. USA* 78:1527); gpt, which confers resistance to mycophenolic acid (Mulligan & Berg, 1981, *Proc. Natl. Acad. Sci. USA* 78:2072); neo, which confers resistance to the aminoglycoside G-418 (Colberre-Garapin *et al*., 1981, *J. Mol. Biol.* 150:1); and hygro, which confers resistance to hygromycin (Santerre *et al*., 1984, *Gene* 30:147).

### 5.2.2 ANTIBODIES

The methods of the present invention encompass the use of antibodies or fragments thereof capable of specifically or selectively recognizing one or more HVEM or LIGHT polypeptide epitopes or epitopes of conserved variants or peptide fragments of the HVEM or LIGHT polypeptides. Such antibodies may include, but are not limited to, polyclonal antibodies, monoclonal antibodies (mAbs), humanized or chimeric antibodies, single chain antibodies, Fab fragments, F(ab')₂ fragments, Fv fragments, fragments produced by a Fab expression library, anti-idiotypic (anti-Id) antibodies, and epitope-binding fragments of any of the above. In a preferred embodiment, the anti-HVEM or anti-LIGHT antibody used in the present methods binds to the HVEM or LIGHT extracellular domain.

Described herein are methods for the production of antibodies or fragments thereof. Any of such antibodies or fragments thereof may be produced by standard immunological methods or by recombinant expression of nucleic acid molecules encoding the antibody or fragments thereof in an appropriate host organism.

For the production of antibodies against an HVEM or LIGHT polypeptide, various host animals may be immunized by injection with an HVEM or LIGHT polypeptide or peptide. Such host animals may include but are not limited to rabbits, mice, and rats, to name but a few. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and *Corynebacterium parvum*.

Polyclonal antibodies are heterogeneous populations of antibody molecules derived from the sera of animals immunized with an antigen, such as an HVEM or LIGHT polypeptide, or an antigenic functional derivative thereof. For the production of polyclonal antibodies, host animals such as those described above, may be immunized by injection with HVEM or LIGHT polypeptide supplemented with adjuvants as also described above.

Monoclonal antibodies, which are homogeneous populations of antibodies to a particular antigen, may be obtained by any technique which provides for the production of antibody molecules by continuous cell lines in culture. These include, but are not limited to, the hybridoma technique of Kohler and Milstein, (1975, *Nature* 256:495; and U.S. Patent No. 4,376,110), the human B-cell hybridoma technique (Kosbor *et al*., 1983, *Immunology Today* 4:72; Cole *et al*., 1983, *Proc. Natl. Acad. Sci. USA* 80:2026), and the EBV-hybridomatechnique (Cole *et al*., 1985, *Monoclonal Antibodies And Cancer Therapy*, Alan R. Liss, Inc., pp. 77). Such antibodies may be of any immunoglobulin class including IgG, IgM, IgE, IgA, IgD and any subclass thereof. The hybridoma producing the mAb of this invention may be cultivated *in vitro* or *in vivo*. Production of high titers of mAbs *in vivo* makes this the presently preferred method of production.

Techniques developed for the production of "chimeric antibodies" (Morrison et al., 1984, Proc. Natl. Acad. Sci., 81, 6851-6855; Neuberger et al., 1984, Nature 312, 604-608; Takeda et al., 1985, Nature 314, 452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. (See, *e.g*., Cabilly et al., U.S. Patent No. 4,816,567; and Boss et al., U.S. Patent No. 5,816,397). The invention thus contemplates chimeric antibodies that are specific or selective for an HVEM or LIGHT polypeptide.

Examples of techniques that have been developed for the production of humanized antibodies are known in the art. (See, *e.g*., Queen, U.S. Patent No. 5,585,089 and Winter, U.S. Patent No. 5,225,539.) An immunoglobulin light or heavy chain variable region consists of a "framework" region interrupted by three hypervariable regions, referred to as complementarity-determining regions (CDRs). The extent of the framework region and CDRs have been precisely defined (see, "Sequences of Proteins of Immunological Interest", Kabat, E. et al., U.S. Department of Health and Human Services (1983). Briefly, humanized antibodies are antibody molecules from non-human species having one or more CDRs from the non-human species and framework regions from a human immunoglobulin molecule. The invention includes the use of humanized antibodies that are specific or selective for an HVEM or LIGHT polypeptide in the methods and compositions of the invention.

Completely human HVEM or LIGHT antibodies are particularly desirable for therapeutic treatment of human patients. Such antibodies can be produced, for example, using transgenic mice which are incapable of expressing endogenous immunoglobulin heavy and light chains genes, but which can express human heavy and light chain genes. The transgenic mice are immunized in the normal fashion with a selected antigen, *e.g*., all or a portion of an HVEM or LIGHT protein. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies. For an overview of this technology for producing human antibodies, see Lonberg and Huszar (1995, *Int. Rev. Immunol*. 13:65-93). For a detailed discussion of this technology for producing human antibodies and human monoclonal antibodies and protocols for producing such antibodies, *see, e.g*., U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA), can be engaged to provide human antibodies directed against a selected HVEM or LIGHT antigen using technology similar to that described above.

Completely human antibodies which recognize a selected epitope can be generated using a technique referred to as "guided selection." In this approach a selected non-human monoclonal antibody, *e.g.*, a murine antibody, is used to guide the selection of a completely human antibody recognizing the same epitope (Jespers *et al*., 1994, *Bio*/*technology* 12:899-903).

The methods of the invention encompasses the use of an antibody or derivative thereof comprising a heavy or light chain variable domain, said variable domain comprising (a) a set of three complementarity-determining regions (CDRs), in which said set of CDRs are from a monoclonal antibody to an HVEM or LIGHT polypeptide, most preferably to the HVEM or LIGHT extracellular domain, and (b) a set of four framework regions, in which said set of framework regions differs from the set of framework regions in the monoclonal antibody, and in which said antibody or derivative thereof immunospecifically binds to the HVEM or LIGHT polypeptide. Preferably, the set of framework regions is from a human monoclonal antibody, *e.g*., a human monoclonal antibody that does not bind the polypeptide encoded for by the HVEM or LIGHT gene sequence.

Phage display technology can be used to increase the affinity of an antibody to an HVEM or LIGHT polypeptide. This technique would be useful in obtaining high affinity antibodies to an HVEM or LIGHT polypeptide that could be used in the combinatorial methods of the invention. The technology, referred to as affinity maturation, employs mutagenesis or CDR walking and re-selection using the HVEM or LIGHT polypeptide antigen to identify antibodies that bind with higher affinity to the antigen when compared with the initial or parental antibody (*see, e.g*., Glaser *et al*., 1992, *J. Immunology* 149:3903). Mutagenizing entire codons rather than single nucleotides results in a semi-randomized repertoire of amino acid mutations. Libraries can be constructed consisting of a pool of variant clones each of which differs by a single amino acid alteration in a single CDR and which contain variants representing each possible amino acid substitution for each CDR residue. Mutants with increased binding affinity for the antigen can be screened by contacting the immobilized mutants with labeled antigen. Any screening method known in the art can be used to identify mutant antibodies with increased avidity to the antigen *(e.g.,* ELISA) (See Wu *et al*., 1998, *Proc Natl. Acad Sci. USA* 95:6037; Yelton *et al*., 1995, *J. Immunology* 155:1994). CDR walking which randomizes the light chain is also possible (*See* Schier *et al*., 1996, *J*. *Mol. Bio*. 263:551).

Alternatively, techniques described for the production of single chain antibodies (U.S. Patent 4,946,778; Bird, 1988, *Science* 242:423; Huston *et al*., 1988, *Proc. Natl. Acad. Sci. USA* 85:5879; and Ward *et al*., 1989, *Nature* 334:544) can be adapted to produce single chain antibodies against HVEM or LIGHT polypeptides. Single chain antibodies are formed by linking the heavy and light chain fragments of the Fv region via an amino acid bridge, resulting in a single chain polypeptide. Techniques for the assembly of functional Fv fragments in *E. coli* may also be used (Skerra *et al*., 1988, *Science* 242:1038).

The methods of the invention include using an antibody to an HVEM or LIGHT polypeptide, peptide or other derivative, or analog thereof that is a bispecific antibody (see generally, *e.g.,* Fanger and Drakeman, 1995, *Drug News and Perspectives* 8:133-137). Such a bispecific antibody is genetically engineered to recognize both (1) an epitope and (2) one of a variety of "trigger" molecules, *e.g*., Fc receptors on myeloid cells, and CD3 and CD2 on T cells, that have been identified as being able to cause a cytotoxic T-cell to destroy a particular target. Such bispecific antibodies can be prepared either by chemical conjugation, hybridoma, or recombinant molecular biology techniques known to the skilled artisan.

Antibody fragments which recognize specific epitopes may be generated by known techniques. For example, such fragments include but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries maybe constructed (Huse *et al*., 1989, *Science* 246:1275-1281) to allow rapid and easy identification of monoclonal Fab fragments with the desired specificity.

### 5.2.3 LIGHT OR HVEM ANTISENSE COMPOUNDS

The present invention encompasses the use of LIGHT and HVEM antisense nucleic acid molecules, *i.e*., molecules which are complementary to a sense nucleic acid encoding LIGHT and HVEM, respectively, *e.g*., complementary to the coding strand of a double-stranded cDNA molecule or complementary to an mRNA sequencem as HVEM-LIGHT inhibitors. Accordingly, an antisense nucleic acid can hydrogen bond to a sense nucleic acid. The antisense nucleic acid can be complementary to an entire coding strand, or to only a portion thereof, *e.g*., all or part of the protein coding region (or open reading frame) of HVEM or LIGHT. An antisense nucleic acid molecule can be antisense to all or part of a non-coding region of the coding strand of a nucleotide sequence encoding HVEM or LIGHT. The non-coding regions ("5' and 3' untranslated regions") are the 5' and 3' sequences which flank the coding region and are not translated into amino acids.

An antisense oligonucleotide can be, for example, about 5, 10, 15, 20, 25, 30, 35, 40, 45 or 50 nucleotides or more in length. An antisense nucleic acid of the invention can be constructed using chemical synthesis and enzymatic ligation reactions using procedures known in the art. For example, an antisense nucleic acid (*e.g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g.*, phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i.e*., RNA transcribed from the inserted nucleic acid will be of an antisense orientation to an HVEM or LIGHT nucleic acid).

The antisense nucleic acid molecules of the invention are typically administered to a subject or generated *in situ* such that they hybridize with or bind to cellular mRNA and/or genomic DNA encoding HVEM or LIGHT to thereby inhibit expression, *e.g*., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of HVEM or LIGHT antisense nucleic acid molecules includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, *e.g*., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

An HVEM or LIGHT antisense nucleic acid molecule can be an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) *Nucleic Acids Res.* 15:6625-6641). The antisense nucleic acid molecule can also comprise a 2'-o-methylribonucleotide (Inoue et al. (1987) *Nucleic Acids Res.* 15:6131-6148) or a chimeric RNA-DNA analogue (Inoue et al. (1987) *FEBS Lett*. 215:327-330).

The invention also encompasses ribozymes. Ribozymes are catalytic RNA molecules with ribonuclease activity which are capable of cleaving a single-stranded nucleic acid, such as an mRNA, to which they have a complementary region. Thus, ribozymes (*e.g*., hammerhead ribozymes (described in Haselhoff and Gerlach, (1988), *Nature* 334:585-591)) can be used to catalytically cleave HVEM or LIGHT mRNA transcripts to thereby inhibit translation of the HVEM or LIGHT protein encoded by the mRNA. A ribozyme having specificity for an HVEM or LIGHT nucleic acid molecule can be designed based upon the nucleotide sequence of the HVEM or LIGHT cDNAs disclosed herein. For example, a derivative of a *Tetrahymena* L-19 IVS RNA can be constructed in which the nucleotide sequence of the active site is complementary to the nucleotide sequence to be cleaved in a Cech et al. U.S. Patent No. 4,987,071; and Cech et al. U.S. Patent No. 5,116,742. Alternatively, an mRNA encoding a polypeptide of the invention can be used to select a catalytic RNA having a specific ribonuclease activity from a pool of RNA molecules. *See*, *e.g.,* Bartel and Szostak (1993) *Science* 261:1411-1418.

The invention also encompasses nucleic acid molecules which form triple helical structures. For example, expression of HVEM or LIGHT can be inhibited by targeting nucleotide sequences complementary to the regulatory region of the gene encoding HVEM or LIGHT, respectively (*e.g*., the promoter and/or enhancer), to form triple helical structures that prevent transcription of the gene in target cells. *See generally* Helene (1991) *Anticancer Drug Des*. 6(6):569-84; Helene (1992) *Ann. N.Y. Acad. Sci.* 660:27-36; and Maher (1992) *Bioassays* 14(12):807-15.

In various embodiments, the antisense nucleic acid molecules of the invention can be modified at the base moiety, sugar moiety or phosphate backbone to improve, *e.g*., the stability, hybridization, or solubility of the molecule. For example, the deoxyribose phosphate backbone of the nucleic acids can be modified to generate peptide nucleic acids (*see* Hyrup et al. (1996) *Bioorganic & Medicinal Chemistry* 4(1): 5-23). As used herein, the terms "peptide nucleic acids" or "PNAs" refer to nucleic acid mimics, *e.g*., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup et al. (1996), *supra;* Perry-O'Keefe et al. (1996) *Proc. Natl. Acad. Sci. USA* 93: 14670-675.

In another embodiment, PNAs can be modified, *e.g*., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras can be generated which may combine the advantageous properties of PNA and DNA. Such chimeras allow DNA recognition enzymes, *e.g*., RNAse H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup (1996), *supra*). The synthesis of PNA-DNA chimeras can be performed as described in Hyrup (1996), *supra,* and Finn et al. (1996) *Nucleic Acids Res.* 24(17):3357-63. For example, a DNA chain can be synthesized on a solid support using standard phosphoramidite coupling chemistry and modified nucleoside analogs. Compounds such as 5'-(4-methoxytrityl)amino-5'-deoxy-thymidine phosphoramidite can be used as a link between the PNA and the 5' end of DNA (Mag et al. (1989) *Nucleic Acids Res.* 17:5973-88). PNA monomers are then coupled in a stepwise manner to produce a chimeric molecule with a 5' PNA segment and a 3' DNA segment (Finn et al. (1996) *Nucleic Acids Res.* 24(17):3357-63). Alternatively, chimeric molecules can be synthesized with a 5' DNA segment and a 3' PNA segment (Peterser et al. (1975) *Bioorganic Med. Chem. Lett.* 5:1119-11124).

In other embodiments, the HVEM or LIGHT antisense oligonucleotide may include other appended groups such as peptides (*e.g*., for targeting host cell receptors *in vivo*), or agents facilitating transport across the cell membrane *(see, e.g.,* Letsinger et al. (1989) *Proc. Natl. Acad. Sci. USA* 86:6553-6556; Lemaitre et al. (1987) *Proc. Natl. Acad. Sci. USA* 84:648-652; PCT Publication No. WO 88/09810) or the blood-brain barrier *(see, e.g.,* PCT Publication No. WO 89/10134). In addition, the antisense oligonucleotides can be modified with hybridization-triggered cleavage agents (*see, e.g*., Krol et al. (1988) *Bio*/*Techniques* 6:958-976) or intercalating agents (*see, e.g*., Zon (1988) *Pharm. Res.* 5:539-549). To this end, the antisense oligonucleotide may be conjugated to another molecule, *e.g*., a peptide, hybridization triggered cross-linking agent, transport agent, hybridization-triggered cleavage agent, etc.

### 5.3 IMMUNOSUPPRESSIVE AGENTS

As described herein, the present invention encompasses the use of immunosuppressive agents in combination with an HVEM-LIGHT inhibitor to prevent or treat allograft rejection. Any immunosuppressive agent known to those of skill in the art may be used. Such an immunosuppressive agent can be a drug or other small molecule, or a protein, including but not limited to an antibody. As used herein, the term "immunosuppressive agent" excludes HVEM-LIGHT inhibitors with immunosuppressive activity.

In certain specific embodiments of the invention, the immunosuppressive agent is cyclosporine, FK506, rapamycin, or prednisone.

In other embodiments, the immunosuppressive agent is a steroid, most preferably a corticosteroid.

In other embodiments, the immunosuppressive agent is an antibody, most preferably an anti-T cell or anti-B cell antibody. In one embodiment, the antibody is an anti-CD 154 antibody. In another embodiment, the antibody is an anti-CD3 antibody such as OKT3. In yet another embodiment, the antibody is an anti-interleukin-2 receptor antibody. Preparation of immunosuppressive antibodies that are suitable for the claimed methods and compositions can be carried out as described *supra* in Section 5.2.2.

In yet other embodiments, the immunosuppressive agent is a protein, for example a CTLA4-Ig fusion protein or a CD40-Ig fusion protein.

In yet other embodiments, the immunosuppressive agent is an antiproliferative agent, such as, but not limited to azathiopurine or mycophenolate moefitil.

In yet other embodiments, the immunosuppressive agent is a purine analog. In one embodiment, the purine analog is methotrexate. In another embodiment, the purine analog mercaptopurine.

### 5.4 GENE THERAPY

In certain embodiments of the present invention, administration of an HVEM-LIGHT inhibitor or an immunosuppressive agent in the form of gene therapy is contemplated. Gene therapy refers to therapy performed by the administration to a subject of an expressed or expressible nucleic acid. In this embodiment of the invention, the nucleic acids produce their encoded protein that mediates a therapeutic or prophylactic effect.

### 5.4.1 NUCLEIC ACIDS ENCODING HVEM-AND LIGHT-BINDING POLYPEPTIDES

The present invention provides nucleic acids encoding dominant negative forms of the HVEM- and LIGHT-binding polypeptides described in Section 5.2.1, *supra*, for use in expression and gene therapy vectors suitable for production or delivery, respectively, of such polypeptides, to a patient in need thereof.

Nucleic acids useful in the gene therapy methods of the present invention encode the minimal domain of an HVEM protein that interacts with LIGHT, or the minimal domain of a LIGHT protein that interacts with HVEM. Such nucleic acids preferably encode soluble, including secreted, HVEM or LIGHT proteins that interfere with endogenous HVEM-LIGHT interactions in the patients to whom they are administered. In a preferred embodiment, a nucleic acid employed in the present gene therapy-based methods encodes a polypeptide consisting essentially of SEQ ID NO:9, SEQ ID NO:10, or SEQ ID NO:11.

Also included are nucleic acids encoding dominant negative forms of HVEM ligands other than LIGHT *(e.g.,* herpesvirus gD protein (SEQ ID NO:12), lymphotoxin-α (SEQ ID NO: 14), as well as nucleic acids encoding dominant negative forms of receptors to which LIGHT binds other than HVEM (*e.g*., the lymphotoxin-β receptor (SEQ ID NO:16), the TR6 receptor (SEQ ID NO:18)).

The present invention further encompasses the use of nucleic acids comprising a region of homology to a nucleic acid encoding the HVEM-binding domain of LIGHT, or the LIGHT-binding domain of HVEM, as long as such a nucleic acid encodes a polypeptide that can bind to HVEM or LIGHT, respectively, and interfere with endogenous HVEM-LIGHT interactions in a patient to whom the polypeptide is administered. In various embodiments, the region of homology is characterized by at least 50%, at least 55%, at least 60%, at least 65%, at least 70%, at least 75%, at least 80%, at least 85%, at least 90%, at least 95% or at least 98% identity with nucleotides consisting essentially of the regions in the HVEM or LIGHT open reading frames encoding the extracellular domains of the proteins. Methods of determining sequence homology are described in Section 5.2.1 above.

The invention also encompasses the use of nucleic acids that (1) hybridize under stringent, moderate or low stringency hybridization conditions to a nucleic acid consisting essentially of the regions in the HVEM or LIGHT open reading frames encoding the extracellular domains of the proteins and (2) encode polypeptides which bind to LIGHT or HVEM, respectively. Preferably, such encoded polypeptides do not comprise a transmembrane domain.

By way of example and not limitation, procedures using such conditions of low stringency for regions of hybridization of over 90 nucleotides are as follows *(see also* Shilo and Weinberg, 1981, Proc. Natl. Acad. Sci. U.S.A. 78,:6789-6792). Filters containing DNA are pretreated for 6 hours at 40°C in a solution containing 35% formamide, 5X SSC, 50 mM Tris-HCl (pH 7.5), 5 mM EDTA, 0.1% PVP, 0.1% Ficoll, 1% BSA, and 500 µg/ml denatured salmon sperm DNA. Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20 X 10⁶ cpm ³²P-labeled probe is used. Filters are incubated in hybridization mixture for 18-20 h at 40°C, and then washed for 1.5 h at 55°C in a solution containing 2X SSC, 25 mM Tris-HCl (pH 7.4), 5 mM EDTA, and 0.1% SDS. The wash solution is replaced with fresh solution and incubated an additional 1.5 h at 60°C. Filters are blotted dry and exposed for autoradiography. If necessary, filters are washed for a third time at 65-68°C and re-exposed to film. Other conditions of low stringency which may be used are well known in the art (*e.g*., as employed for cross-species hybridizations).

Also, by way of example and not limitation, procedures using such conditions of high stringency for regions of hybridization of over 90 nucleotides are as follows. Prehybridization of filters containing DNA is carried out for 8 h to overnight at 65 °C in buffer composed of 6X SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 h at 65 °C in prehybridization mixture containing 100 µg/ml denatured salmon sperm DNA and 5-20 X 10⁶ cpm of ³²P-labeled probe. Washing of filters is done at 37°C for 1 h in a solution containing 2X SSC, 0.01% PVP, 0.01% Ficoll, and 0.01% BSA. This is followed by a wash in 0.1X SSC at 50°C for 45 min before autoradiography.

Other conditions of high stringency which may be used depend on the nature of the nucleic acid (*e.g*., length, GC content, *etc*.) and the purpose of the hybridization (detection, amplification, *etc*.) and are well known in the art. For example, stringent hybridization of a nucleic acid of approximately 15-40 bases to a complementary sequence in the polymerase chain reaction (PCR) is done under the following conditions: a salt concentration of 50 mM KCl, a buffer concentration of 10 mM Tris-HCl, a Mg²⁺ concentration of 1.5 mM, a pH of 7-7.5 and an annealing temperature of 55-60°C.

Selection of appropriate conditions for moderate stringencies is also well known in the art (*see, e.g*., Sambrook *et al*., 1989, Molecular Cloning, A Laboratory Manual, 2d Ed., Cold Spring Harbor Laboratory Press, Cold Spring Harbor, New York; *see also*, Ausubel *et al*., eds., in the Current Protocols in Molecular Biology series of laboratory technique manuals, © 1987-1997, Current Protocols, © 1994-1997 John Wiley and Sons, Inc.).

The nucleic acids useful in the present methods may be made by any method known in the art. For example, if the nucleotide sequence of the protein is known, a nucleic acid encoding the antibody may be assembled from chemically synthesized oligonucleotides (*e.g*., as described in Kutmeier *et al*., 1994, BioTechniques 17:242), which, briefly, involves the synthesis of overlapping oligonucleotides containing portions of the sequence encoding the protein, annealing and ligating of those oligonucleotides, and then amplification of the ligated oligonucleotides by PCR.

Alternatively, a nucleic acid that is useful in the present methods may be generated from nucleic acid from a suitable source. If a clone containing a nucleic acid encoding a particular protein is not available, but the sequence of the protein molecule is known, a nucleic acid encoding the protein may be chemically synthesized or obtained from a suitable source (*e.g*., a cDNA library such as an antibody cDNA library or a cDNA library generated from, or nucleic acid, preferably poly A+ RNA, isolated from, any tissue or cells expressing the protein.

Further, a nucleic acid that is useful in the present methods may be manipulated using methods well known in the art for the manipulation of nucleotide sequences, *e.g*., recombinant DNA techniques, site directed mutagenesis, PCR, *etc.* (see, for example, the techniques described in Sambrook *et al*., 1990, Molecular Cloning, A Laboratory Manual, 2d Ed., Cold Spring Harbor Laboratory, Cold Spring Harbor, NY and Ausubel *et al*., eds., 1998, Current Protocols in Molecular Biology, John Wiley & Sons, NY, which are both incorporated by reference herein in their entireties), to generate antibodies having a different amino acid sequence, for example to create amino acid substitutions, deletions, and/or insertions.

### 5.4.2 GENE THERAPY METHODS

Any of the methods for gene therapy available in the art can be used In the methods and compositions of the present invention. Exemplary methods are described below.

For general reviews of the methods of gene therapy, *see,* Goldspiel *et al*., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, 1993, Science 260:926-932; Morgan and Anderson, 1993, Ann. Rev. Biochem. 62:191-217; May, 1993, TIBTECH 1, 1(5):155-215. Methods commonly known in the art of recombinant DNA technology which can be used are described in Ausubel *et al*. (eds.), Current Protocols in Molecular Biology, John Wiley & Sons, NY (1993); and Kriegler, Gene Transfer and Expression, A Laboratory Manual, Stockton Press, NY (1990).

In a preferred aspect, the therapeutic comprises nucleic acid sequences encoding an HVEM-LIGHT inhibitor and/or an immunosuppressive agent, said nucleic acid sequences being part of expression vectors that express the HVEM-LIGHT inhibitor and/or immunosuppressive agent in a suitable host. In particular, such nucleic acid sequences have promoters operably linked to the HVEM-LIGHT inhibitor or immunosuppressive agent coding region, said promoter being inducible or constitutive, and, optionally, tissue-specific. In another particular embodiment, nucleic acid molecules are used in which the HVEM-LIGHT inhibitor or immunosuppressive agent coding sequences and any other desired sequences are flanked by regions that promote homologous recombination at a desired site in the genome, thus providing for intrachromosomal expression of the HVEM-LIGHT inhibitor or immunosuppressive agent (Koller and Smithies, 1989, Proc. Natl. Acad. Sci. USA 86:8932-8935; Zijlstra *et al*., 1989, Nature 342:435-438.

Delivery of the nucleic acids into a patient may be either direct, in which case the patient is directly exposed to the nucleic acid or nucleic acid-carrying vectors, or indirect, in which case, cells are first transformed with the nucleic acids *in vitro*, then transplanted into the patient. These two approaches are known, respectively, as *in vivo* or *ex vivo* gene therapy.

In a specific embodiment, the nucleic acid sequences are directly administered *in vivo*, where it is expressed to produce the encoded product. This can be accomplished by any of numerous methods known in the art, for example by constructing them as part of an appropriate nucleic acid expression vector and administering the vector so that the nucleic acid sequences become intracellular. Gene therapy vectors can be administered by infection using defective or attenuated retrovirals or other viral vectors (*see, e.g*., U.S. Patent No. 4,980,286); direct injection of naked DNA; use of microparticle bombardment (*e.g*., a gene gun; Biolistic, Dupont); coating with lipids or cell-surface receptors or transfecting agents; encapsulation in liposomes, microparticles, or microcapsules; administration in linkage to a peptide which is known to enter the nucleus; administration in linkage to a ligand subject to receptor-mediated endocytosis (*see, e.g*., Wu and Wu, 1987, J. Biol. Chem. 262:4429-4432) (which can be used to target cell types specifically expressing the receptors); *etc*. In another embodiment, nucleic acid-ligand complexes can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor *(see, e.g.,* PCT Publications WO 92/06 180; WO 92/22635; W092/20316; W093/14188, and WO 93/20221). Alternatively, the nucleic acid can be introduced intracellularly and incorporated within host cell DNA for expression by homologous recombination (Koller and Smithies, 1989, Proc. Natl. Acad. Sci. USA 86:8932-8935; Zijlstra *et al*., 1989, Nature 342:435-438).

In a specific embodiment, viral vectors that contain nucleic acid sequences encoding an HVEM-LIGHT inhibitor or immunosuppressive agent are used. For example, a retroviral vector can be used (see Miller *et al*., 1993, Meth. Enzymol. 217:581-599). These retroviral vectors contain the components necessary for the correct packaging of the viral genome and integration into the host cell DNA. The nucleic acid sequences encoding the HVEM-LIGHT inhibitor or immunosuppressive agent to be used in gene therapy are cloned into one or more vectors, thereby facilitating delivery of the gene into a patient. More detail about retroviral vectors can be found in Boesen *et al*., 1994, Biotherapy 6:29 1-302, which describes the use of a retroviral vector to deliver the mdr 1 gene to hematopoietic stem cells in order to make the stem cells more resistant to chemotherapy. Other references illustrating the use of retroviral vectors in gene therapy are: Clowes *et al*., 1994, J. Clin. Invest. 93:644-651; Klein *et al*., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

### 5.4.2.1 CELL THERAPY

One approach to gene therapy encompassed by the present methods and compositions involves transferring a gene, *e.g.,* an HVEM-LIGHT inhibitor or immunosuppressive agent gene, to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a patient.

In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell mediated gene transfer, spheroplast fusion, *etc*. Numerous techniques are known in the art for the introduction of foreign genes into cells (*see, e.g*., Loeffler and Behr, 1993, Meth. Enzymol. 217:599-618; Cohen *et al*., 1993, Meth. Enzymol. 217:618-644; Cline, 1985, Pharmac. Ther. 29:69-92) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a patient by various methods known in the art. Recombinant blood cells (*e.g*., hematopoietic stem or progenitor cells) are preferably administered intravenously. The amount of cells envisioned for use depends on the desired effect, patient state, *etc*., and can be determined by one skilled in the art.

Cells into which a nucleic acid can be introduced for purposes of gene therapy encompass any desired, available cell type, and include but are not limited to fibroblasts; blood cells such as T lymphocytes, B lymphocytes, monocytes, macrophages, neutrophils, eosinophils, megakaryocytes, granulocytes; various stem or progenitor cells, in particular hematopoietic stem or progenitor cells, *e.g*., as obtained from bone marrow, umbilical cord blood, peripheral blood, fetal liver, *etc*.

In a preferred embodiment, the cell used for gene therapy is autologous to the patient.

In an embodiment in which recombinant cells are used in gene therapy, nucleic acid sequences encoding an HVEM-LIGHT inhibitor or immunosuppressive agent are introduced into the cells such that they are expressible by the cells or their progeny, and the recombinant cells are then administered *in vivo* for therapeutic effect. In a specific embodiment, stem or progenitor cells are used. Any stem and/or progenitor cells which can be isolated and maintained *in vitro* can potentially be used in accordance with this embodiment of the present invention (see *e.g.* PCT Publication WO 94/08598; Stemple and Anderson, 1992, Cell 71:973-985; Rheinwald, 1980, Meth. Cell Bio. 21A:229; and Pittelkow and Scott, 1986, Mayo Clinic Proc. 61:771).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

### 5.5 ASSAYS FOR HVEM-LIGHT INHIBITORS

In addition to the LIGHT-HVEM described herein, novel HVEM-LIGHT inhibitors, *i.e.,* agents that interfere with the HVEM-LIGHT interaction, can be identified by the methods described below.

The basic principle of the assay systems used to identify HVEM-LIGHT inhibitors involves preparing a reaction mixture containing at least the HVEM-binding portion of LIGHT and the LIGHT-binding portion of HVEM under conditions (referred to in this section as the LIGHT protein and the HVEM protein, respectively) and for a time sufficient to allow the two to interact and bind, thus forming a complex. In order to test a compound for inhibitory activity, the reaction mixture is prepared in the presence and absence of the test compound. The test compound can be initially included in the reaction mixture, or can be added at a time subsequent to the addition of the HVEM and LIGHT proteins. Control reaction mixtures are incubated without the test compound or with a placebo. The formation of any complexes between the HVEM and LIGHT is then detected. The formation of a complex in the control reaction, but not in the reaction mixture containing the test compound, indicates that the compound interferes with the HVEM-LIGHT interaction.

The assay for compounds that interfere with the interaction of HVEM and LIGHT can be conducted in a heterogeneous or homogeneous format. Heterogeneous assays involve anchoring either the HVEM or LIGHT protein onto a solid phase and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between LIGHT and HVEM, *e.g.*, by competition, can be identified by conducting the reaction in the presence of the test substance; *i.e*., by adding the test substance to the reaction mixture prior to or simultaneously with the HVEM and LIGHT proteins. Alternatively, test compounds that disrupt preformed complexes, e.g. compounds with higher binding constants that displace one of the components from the complex, can be tested by adding the test compound to the reaction mixture after complexes have been formed. The various formats are described briefly below.

In a heterogeneous assay system, either the HVEM or LIGHT protein, is anchored onto a solid surface, while the non-anchored species is labeled, either directly or indirectly. In practice, microtiter plates are conveniently utilized. The anchored species can be immobilized by non-covalent or covalent attachments. Non-covalent attachment can be accomplished simply by coating the solid surface with a solution of the HVEM or LIGHT protein and drying. Alternatively, an immobilized antibody specific for the species to be anchored can be used to anchor the species to the solid surface. The surfaces can be prepared in advance and stored.

In order to conduct the assay, the partner of the immobilized species is exposed to the coated surface with or without the test compound. After the reaction is complete, unreacted components are removed (*e.g*., by washing) and any complexes formed will remain immobilized on the solid surface. The detection of complexes anchored on the solid surface can be accomplished in a number of ways. Where the non-immobilized species is pre-labeled, the detection of label immobilized on the surface indicates that complexes were formed. Where the non-immobilized species is not pre-labeled, an indirect label can be used to detect complexes anchored on the surface; *e.g*., using a labeled antibody specific for the initially non-immobilized species (the antibody, in turn, can be directly labeled or indirectly labeled with a labeled anti-Ig antibody). Depending upon the order of addition of reaction components, test compounds which inhibit complex formation or which disrupt preformed complexes can be detected.

Alternatively, the reaction can be conducted in a liquid phase in the presence or absence of the test compound, the reaction products separated from unreacted components, and complexes detected; *e.g*., using an immobilized antibody specific for one of the binding components to anchor any complexes formed in solution, and a labeled antibody specific for the other partner to detect anchored complexes. Again, depending upon the order of addition of reactants to the liquid phase, test compounds which inhibit complex or which disrupt preformed complexes can be identified.

In an alternate embodiment of the invention, a homogeneous assay can be used. In this approach, a preformed complex of the HVEM and LIGHT proteins is prepared in which either the HVEM or LIGHT protein is labeled, but the signal generated by the label is quenched due to complex formation (see, *e.g*., U.S. Patent No. 4,109,496 by Rubenstein which utilizes this approach for immunoassays). The addition of a test substance that competes with and displaces one of the species from the preformed complex will result in the generation of a signal above background. In this way, test substances which disrupt HVEM-LIGHT interaction can be identified.

In a particular embodiment, the target gene product can be prepared for immobilization using recombinant DNA techniques known to those of skill in the art. For example, the HVEM or LIGHT protein can be fused to a glutathione-S-transferase (GST) gene using a fusion vector, such as pGEX-5X-1, in such a manner that its binding activity is maintained in the resulting fusion protein. The binding partner (*i.e*., the LIGHT or HVEM protein, respectively) can be purified and used to raise a monoclonal antibody, using methods routinely practiced in the art and described above. This antibody can be labeled with the radioactive isotope ¹²⁵I, for example, by methods routinely practiced in the art. In a heterogeneous assay, *e.g.,* the GST-LIGHT or GST-HVEM fusion protein can be anchored to glutathione-agarose beads. The HVEM or LIGHT protein, respectively, can then be added in the presence or absence of the test compound in a manner that allows interaction and binding to occur. At the end of the reaction period, unbound material can be washed away, and the labeled monoclonal antibody can be added to the system and allowed to bind to the complexed components. The interaction between HVEM and LIGHT can be detected by measuring the amount of radioactivity that remains associated with the glutathione-agarose beads. A successful inhibition of the interaction by the test compound will result in a decrease in measured radioactivity.

Alternatively, the GST-HVEM or GST-LIGHT fusion protein and its binding partner (*i.e*., LIGHT or HVEM protein, respectively) can be mixed together in liquid in the absence of the solid glutathione-agarose beads. The test compound can be added either during or after the species are allowed to interact. This mixture can then be added to the glutathione-agarose beads and unbound material is washed away. Again the extent of inhibition of the HVEM-LIGHT interaction can be detected by adding the labeled antibody and measuring the radioactivity associated with the beads.

In one embodiment of the foregoing methods, the test compound is a peptide fragment that corresponds to the extracellular portion of HVEM or LIGHT, thereby allowing the identification of small HVEM-LIGHT inhibitor peptides that can be produced synthetically instead of recombinantly for use in the present methods and compositions.

### 5.5.1 CELL AND ANIMAL-BASED MODEL SYSTEMS

Described herein are cell- and animal-based systems which act as models for immune disorders of the present invention. Such model systems can be used to test compounds identified, *e.g.,* using the *in vitro* assays described in Section 5.4.1, above, for their ability and/or effectiveness in treating (*e.g*., ameliorating or modulating symptoms of) immune-related disorders. Thus, the animal- and cell-based models of the invention can be used to identify drugs, pharmaceuticals, therapies and interventions which, when administered to a patient with an immunosuppressive agent, can be effective in treating the immune disorders of the present invention. In addition, as described in detail, below, such animal models can be used to determine the LD₅₀ and the ED₅₀ in animal subjects, and such data can be used to determine the *in vivo* efficacy of potential immune disorder treatments.

### 5.5.1.1 ANIMAL-BASED SYSTEMS

Animal-based model systems of immune disorders can include both non-recombinant animals as well as recombinantly engineered transgenic animals.

Animal models for TH cell subpopulation-related disorders can include, for example, genetic models. For example, such animal models can include Leishmania resistance models, experimental allergic encephalomyelitis models and (BALB/c Cr x DBA/2Cr) F1 mice. These latter mice develop a fatal disseminated disease by systemic infection with virulent *Candida albicans* associated with strong TH2-like responses. Well known mouse models for asthma can be utilized to study the amelioration of symptoms caused in immune disorders, such as allergy and asthma, that are associated with a strong TH2 or TH2-like response. (See, for example, Lukacs *et al*., 1994, Am. J. Resp. Cell Mol. Biol. 10:526-532; Gavett *et al*., 1994, Am. J. Resp. Cell Mol. Biol. 10:587-593.) Further, the animal model, murine acquired immunodeficiency syndrome (MAIDS; Kanagawa *et al*., 1993, Science 262:240; Makino *et al*., 1990, J. Imm. 144:4347) can be used for such studies.

Alternatively, such well known animal models as SCIDhu mice (see for example, H. Kaneshima *et al*., 1994, Curr. Opin. Imm. 6:327-333) which represents an *in vivo* model of the human hematolymphoid system, can be utilized. Further, the RAG-2-deficient blastocyst complementation technique (J. Chen *et al*., 1993, Proc. Natl. Acad. Sci. USA 90:4528-4532; Y. Shinkai et al., 1992, *Cell 68*:855-867) can be utilized to produce mice containing, for example, humanized lymphocytes and/or which express target gene sequences. Still further, targeting techniques directed specifically to T cells, for example, the technique of Gu *et al*. (1994, Science 265:103-106) can be utilized to produce animals containing transgenes in only T cell populations.

Further, animal models such as the adoptive transfer model described, *e.g.,* in L. Cohn *et al*., 1997, J. Exp. Med. 186:1737-1747) below, can be used. In such an animal system, aeroallergen provocation of TH1 or TH2 recipient mice results in TH effector cell migration to the airways and is associated with an intense neutrophilic (TH1) and eosinophilic (TH2) lung mucosal inflammatory response. The animal model represents an accepted model for asthma.

In a preferred embodiment, the animal model for testing the efficacy of the HVEM-LIGHT inhibitor, *e.g.,* an inhibitor identified by the *in vitro-based* methods described above, is an allograft rejection, most preferably a cardiac allograft rejection, murine model.

Animals models for other immune disorders are known in the art. TH1-related disorder symptoms can include, for example, those associated with chronic inflammatory diseases and disorders, such as Crohn's disease, reactive arthritis (including but not limited to Lyme disease), insulin-dependent diabetes, organ-specific autoimmunity, (including but not limited to multiple sclerosis, Hashimoto's thyroiditis and Grave's disease), contact dermatitis, psoriasis, graft rejection, graft versus host disease and sarcoidosis to name a few. TH2--related disorder symptoms can include, but are not limited to, those associated with atopic conditions such as asthma and allergy such as allergic rhinitis, gastrointestinal allergies, (including but not limited to food allergies); eosinophilia; conjunctivitis; glomerular nephritis; certain pathogen susceptibilities such as helminthic (*e.g.*, leishmaniasis); and certain viral infections, including HIV, and bacterial infections (for example, tuberculosis and lepromatous leprosy).

Additionally, cellular phenotypes characteristic of the immune disorders of the invention can be assayed for efficacy of the HVEM-LIGHT inhibitor and immunosuppressive agent combinatorial therapy. Such cellular phenotypes can include, for example, cytokine expression profiles, which, are assayed for reversion from the disease state to the normal states upon administration of the HVEM-LIGHT inhibitor and immunosuppressive agent in accordance with the methods of the invention, for example as described in Section 6.2 below.

### 5.5.1.2 CELL-BASED ASSAYS

Cell populations which endogenously or recombinantly express HVEM and LIGHT proteins can be utilized to identify or validate potential HVEM-LIGHT inhibitors. Cellular phenotypes which can indicate inhibition of the LIGHT-HVEM interaction can include, for example, an inhibition of cytokine or cell surface marker expression associated with LIGHT-HVEM signaling.

In order to recombinantly express a LIGHT or HVEM protein, the LIGHT or HVEM open reading frame, respectively, can be ligated to a regulatory sequence which is capable of driving gene expression in the cell type of interest. Such regulatory regions will be well known to those of skill in the art, and can be utilized in the absence of undue experimentation. Transfection of the LIGHT or HVEM expression construct can be accomplished by utilizing standard techniques. See, for example, Ausubel, 1989, *supra.* Transfected cells should be evaluated for the presence of the recombinant target gene sequences, for expression and accumulation of target gene mRNA, and for the presence of recombinant target gene protein production.

### 5.6 PHARMACEUTICAL PREPARATIONS AND METHODS OF ADMINISTRATION

The immunosuppressive agents and HVEM-LIGHT inhibitors that are useful in the present methods and compositions, such as those described herein, can be administered to a patient in amounts effective to treat or prevent allograft rejection. As used herein, "amounts effective to treat or prevent allograft rejection" refers to the combined amount of HVEM-LIGHT inhibitor and immunosuppressive agent.

### 5.6.1 EFFECTIVE DOSE

The present invention provides the benefit of administration of reduced amounts of the HVEM-LIGHT inhibitor and the immunosuppressive agent relative what would be required for each of the compounds alone to achieve a therapeutic or prophylactic effect, thereby lowering the toxicity associated with administration of such compounds. Toxicity and therapeutic efficacy of compounds can be determined by standard pharmaceutical procedures in cell cultures or experimental animals, *e.g.*, for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects can be used, care should be taken to design a delivery system that targets such compounds to the site of affected tissue in order to minimize potential damage to unaffected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (*i.e*., the concentration of the test compound which achieves a half-maximal inhibition of symptoms) as determined in cell culture. Such information can be used to more accurately determine useful doses in humans. Levels in plasma can be measured by any technique known in the art, for example, by high performance liquid chromatography.

For compounds whose therapeutic dose in single therapy regimens has been determined, it is preferable to use dosages at the lower end of or below the determined therapeutic dose, although higher doses can be used, for example where single therapy is not successful. For example, the therapeutic dose of cyclosporine A for treating graft rejection in humans is in the range of 2 to 6 mg/kg/day. Thus, in the present methods and compositions, cyclosporine A can be used in amounts of, for example, 0.5-10 mg/kg/day, more preferably 1-5 mg/kg/day, and most preferably 2-3 mg/kg/day.

### 5.6.2 FORMULATIONS AND USE

The invention relates to pharmaceutical compositions and methods of use thereof for preventing or treating allograft rejection. Such pharmaceutical compositions can be formulated in a conventional manner using one or more physiologically acceptable carriers or excipients. In one embodiment of the present invention, the HVEM-LIGHT inhibitor and the immunosuppressive agent are formulated into one pharmaceutical composition, most preferably in amounts that are effective to treat or prevent an immune disorder such as allograft rejection.

Thus, the compounds and their physiologically acceptable salts and solvents can be formulated for systemic administration or local administration at the site of immune dysfunction. Further, the compounds can be formulated for administration by inhalation or insufflation (either through the mouth or the nose) or oral, buccal, parenteral or rectal administration.

For oral administration, the pharmaceutical compositions can take the form of, for example, tablets or capsules prepared by conventional means with pharmaceutically acceptable excipients such as binding agents (*e.g*., pregelatinised maize starch, polyvinylpyrrolidone or hydroxypropyl methylcellulose); fillers (*e.g.*, lactose, microcrystalline cellulose or calcium hydrogen phosphate); lubricants (*e.g*., magnesium stearate, talc or silica); disintegrants (*e.g*., potato starch or sodium starch glycolate); or wetting agents (*e.g*., sodium lauryl sulphate). The tablets can be coated by methods well known in the art. Liquid preparations for oral administration can take the form of, for example, solutions, syrups or suspensions, or they can be presented as a dry product for constitution with water or other suitable vehicle before use. Such liquid preparations can be prepared by conventional means with pharmaceutically acceptable additives such as suspending agents (*e.g*., sorbitol syrup, cellulose derivatives or hydrogenated edible fats); emulsifying agents (*e.g*., lecithin or acacia); non-aqueous vehicles (*e.g*., almond oil, oily esters, ethyl alcohol or fractionated vegetable oils); and preservatives (*e.g*., methyl or propyl-p-hydroxybenzoates or sorbic acid). The preparations can also contain buffer salts, flavoring, coloring and sweetening agents as appropriate.

Preparations for oral administration can be suitably formulated to give controlled release of the active compound.

For buccal administration the compositions can take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of, e.g., gelatin for use in an inhaler or insufflator can be formulated containing a powder mix of the compound and a suitable powder base such as lactose or starch.

The compounds can be formulated for parenteral administration (*i.e*., intravenous or intramuscular) by injection, via, for example, bolus injection or continuous infusion. Formulations for injection can be presented in unit dosage form, *e.g*., in ampoules or in multi-dose containers, with an added preservative. The compositions can take such forms as suspensions, solutions or emulsions in oily or aqueous vehicles, and can contain formulatory agents such as suspending, stabilizing and/or dispersing agents. Alternatively, the active ingredient can be in powder form for constitution with a suitable vehicle, *e.g*., sterile pyrogen-free water, before use.

The compounds can also be formulated in rectal compositions such as suppositories or retention enemas, *e.g*., containing conventional suppository bases such as cocoa butter or other glycerides.

In addition to the formulations described previously, the compounds can also be formulated as a depot preparation. Such long acting formulations can be administered by implantation (for example subcutaneously or intramuscularly) or by intramuscular injection. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

### 5.7 KITS

The present invention provides kits for practicing the methods of the present invention. A kit of the invention comprises in one or more containers an HVEM-LIGHT inhibitor, such as those described in Section 5.2, *supra*, or an immunosuppressive agent, such as those described in Section 5.3, *supra*.

The kit of the invention may optionally comprise additional components useful for performing the methods of the invention. By way of example, the kit may comprise pharmaceutical carriers useful for formulating the HVEM-LIGHT inhibitor and/or the immunosuppressive agent. Where the HVEM-LIGHT inhibitor or immunosuppressive agent is administered in the form of cell therapy or gene therapy, suitable cells or gene therapy vectors may also be included. In addition, the kits of the invention may further provide an instructional material which describes performance of the methods of the inveniton, or a notice in the form prescribed by a governmental agency regulating the manufacture, use or sale of pharmaceuticals or biological products, which notice reflects approval by the agency of manufacture, use or sale for human administration.

### 5.8 MURINE HVEM GENE SEQUENCES

The present invention discloses novel murine HVEM nucleic acids and polypeptides. Prior to the present disclosure, the full length murine cDNA and its encoded protein had not been characterized. As shown in FIG. 1, the murine and human sequences share 46% sequence identify and 57% sequence similarity, with the greatest stretch of identity being seven consecutive amino acids at positions 88-94.

### 5.8.1 mHVEM NUCLEIC ACIDS

The present invention provides murine HVEM nucleic acids. The murine HVEM cDNA is represented by SEQ ID NO:3.

In one embodiment, the present invention provides an isolated nucleic acid molecule having a nucleotide sequence which is at least 65%, 70%, 75%, 80%, 85%, 90%, 95% or 98% identical to the nucleotide sequence of SEQ ID NO:3 or a complement thereof as determined using the BLAST algorithm. In another embodiment, the present invention provides an isolated nucleic acid molecule comprising at least 15, 20, 30, 40 or 50 nucleotide residues and having a nucleotide sequence identical to at least 15, 20, 30, 40 or 50 consecutive nucleotide residues of residues 1-424 of SEQ ID NO:3 or a complement thereof. In another embodiment, the present invention provides an isolated nucleic acid molecule which encodes a polypeptide comprising the amino acid sequence of SEQ ID NO:4. In yet another embodiment, the present invention provides an isolated nucleic acid molecule which encodes a fragment of a polypeptide comprising the amino acid sequence of any of SEQ ID NO:4, wherein the fragment comprises at least 10, 12, 15, 20, 25, 30, 40 or 50 consecutive amino acid residues of residues 1-80 of SEQ ID NO:4. In yet another embodiment, the present invention provides an isolated nucleic acid molecule which encodes a naturally occurring allelic variant of a polypeptide comprising the amino acid sequence of SEQ ID NO:4, wherein the nucleic acid molecule hybridizes under stringent conditions with a nucleic acid molecule consisting of the nucleotide sequence of any of residues 1-424 of SEQ ID NO:3 or a complement thereof. Preferably, the foregoing nucleic acids encode a polypeptide that has one or more characteristics of mHVEM. Such characteristics include the ability to bind to gD, LIGHT, or Ltα; or the ability to immunospecifically bind to an antibody that immunospecifically binds to mHVEM.

Further, the present invention provides an isolated nucleic acid having the nucleotide sequence of SEQ ID NO:3 or a complement thereof. The present invention also provides an isolated nucleic acid molecule which encodes a polypeptide having the amino acid sequence of any of SEQ ID NO:4 or a complement thereof.

Nucleic acids encoding any of the mHVEM polypeptides described in Section 5.8.2 below are also provided.

In a preferred embodiment, the foregoing nucleic acids further comprise vector nucleic acid sequences and/or nucleic acid sequences encoding a heterologous polypeptide.

The present invention further provides mammalian and non-mammalian host cells containing any of the foregoing mHVEM nucleic acid molecules. The host cell can be a mammalian or non-mammalian host cell.

### 5.8.2 mHVEM POLYPEPTIDES

The present invention provides murine HVEM polypeptides. The murine HVEM open reading frams is represented by SEQ ID NO:4.

The present invention provides an isolated polypeptide comprising a fragment of a polypeptide comprising the amino acid sequence of SEQ ID NO:4, wherein the fragment comprises at least 10, 12, 15, 20, 25, 30, 40 or 50 consecutive amino acid residues of residues 1-80 of SEQ ID NO:4. The present invention further provides an isolated polypeptide comprising naturally occurring allelic variant of a polypeptide comprising the amino acid sequence of any of SEQ ID NO:4, wherein the polypeptide is encoded by a nucleic acid molecule which hybridizes with a nucleic acid molecule consisting of the nucleotide sequence of residues 1-424 of SEQ ID NO:3 or a complement thereof under stringent conditions. Yet further, the present invention provides a polypeptide which is encoded by a nucleic acid molecule comprising a nucleotide sequence which is at least 65%, 70%, 75%, 80%, 85%, 90%, 95% or 98% identical to a nucleic acid consisting of residues 1-424 of SEQ ID NO:3 or a complement thereof as determined using the NBLAST algorithm. Polypeptides encoded by any of the mHVEM nucleic acids described in Section 5.8.1, *supra*, are also provided.

In a specific embodiment, the present invention provides a polypeptide having the amino acid sequence of SEQ ID NO:4.

The present invention yet further provides a polypeptide, preferably an isolated polypeptide, comprising any of the foregoing mHVEM polypeptides fused at its amino or carboxy terminal to heterologous amino acid residues. Suitable fusion partners for mHVEM polypeptides are described in Section 5.2.1.1., *supra*.

The present invention further provides antibodies which selectively bind to murine HVEM polypeptides, but not to human HVEM polypeptides. Such antibodies can be made by the methods described in Section 5.2.2. above.

The present invention further provides methods of making mHVEM polypeptides, comprising culturing a host cell comprising a recombinant nucleic acid that encodes a mHVEM polypeptide operably linked to a promoter under conditions such that the mHVEM polypeptide is expressed.

The following experimental examples are offered by way of illustration and not by way of limitation.

### 6. EXAMPLE: MODULATION OF LIGHT-HVEM COSTIMULATION PROLONGS CARDIAC ALLOGRAFT SURVIVAL

LIGHT, a TNF superfamily member expressed by activated T cells, binds to herpesvirus entry mediator (HVEM) which is constitutively expressed by T cells. LIGHT costimulates T cell activation in a CD28-independent manner, and given interest in regulating the effector functions of T cells in vivo, the role of LIGHT-HVEM costimulation was examined in a murine cardiac allograft rejection model. Normal hearts lacked LIGHT or HVEM mRNA expression, but allografts showed strong expression of both genes from day 3 post-transplant, and in-situ hybridization and immunohistology localized LIGHT and HVEM to infiltrating leukocytes. To test the importance of LIGHT expression on allograft survival, we generated LIGHT-/- mice by homologous recombination. The survival of fully MHC-mismatched cardiac allografts was longer in LIGHT-/- vs. LIGHT+/+ mice (p<0.05), and LIGHT-/- allograft recipients treated with cyclosporin A (CsA) maintained their allografts longer than untreated LIGHT-/- mice (p<0.001) or CsA-treated wild-type (LIGHT+/+) controls (p<0.001). Molecular analyzes showed that the beneficial effects of targeting of LIGHT were accompanied by decreased intragraft expression of IFN-γ, plus IFN-γ-induced chemokine, IP-10, and its receptor, CXCR3. Further evidence in support of a role of this pathway in alloresponses was shown by the beneficial effects on allograft survival in LIGHT+/+ allograft recipients treated with HVEM-Ig plus CsA. The data presented herein indicates that LIGHT/HVEM costimulation is a significant component of the host response leading to cardiac allograft rejection.

### 6.1 MATERIALS AND METHODS

*Generation of LIGHT-*/*- Mice.* A targeting vector was constructed using a 10.5-kb genomic fragment containing exons 1-4 of the LIGHT gene. A 0.8 kb sequence around exon 1, including ATG, was deleted and replaced by pMC1 neo, the targeting vector was linearized and electroporated into ES cells, and LIGHT+/- ES cell clones were selected in media containing G418 and Gancyclovir, as described (Hancock *et al*., 2000, J Exp Med 192:1515-20). The correctly targeted event was screened by Southern blotting, and chimeric mice were derived by blastocyst injection. Offspring of LIGHT+/- mice were crossed to produce LIGHT-/- mice. Mice used in this study were on a B6/129 background.

*Transplantation.* Male 6-week-old BALB/c (H-2^{d}), 129Sv/J (H-2^{b}), C57BL/6 (H-2^{b}) and B6/129 (H-2^{b}) mice were purchased from The Jackson Laboratory and maintained in our specific pathogen-free facility. Heterotopic abdominal cardiac allografting was done with the use of BALB/c donors and B6/129 recipients, as well as in some experiments, pure 129 or B6 recipients, using 6 allografts per experimental group (Hancock *et al*., 1996, Proc Natl Acad Sci USA 93:13967-72); data reported are from BALB->B6/129 unless noted otherwise. Allograft function was monitored twice daily by palpation, and rejection confirmed by laparotomy and histologic examination. Upon harvest at rejection or at the time indicated, mid-ventricular samples were fixed in formalin for light microscopy or snap-frozen in liquid nitrogen and stored at -80 °C for subsequent immunohistology and RNA studies.

*Post-transplant Therapies.* LIGHT-/- or LIGHT+/+ allograft recipients were treated with cyclosporin A (CsA) (Sigma), dissolved in olive oil and administered daily (10 mg/kg i.p.) for 14 d post-transplant. Additional LIGHT+/+ recipients were treated with a mHVEM-human IgG1 (HVEM-Ig) fusion protein, which was generated using the extracellular region of HVEM (Mauri *et al*., 1998, Immunity 8:21-30); recipients received 100 µg of HVEM-Ig daily, from the time of transplantation until day 14.

*RNA Isolation and RT-PCR Analysis of HYEM and LIGHT Expression*. Total RNA was prepared from each recipient's heart and cardiac allograft using guanidine-thiocyanate, and RNA integrity was confirmed by electrophoresis (Chomczynski and Sacchi, 1987, Anal. Biochem. 162:156-9). RNA was reverse transcribed at 45 °C for 60 min, 95 °C for 3 min, and placed on ice. For LIGHT detection, primers (mLIGHTIF, 5-ATGGAGAGTGTGGTACAGCCTTC-3 (SEQ ID NO:20); mLIGHTIR, 5-GACCATGAAAGCTCCGAAATAGG-3 (SEQ ID NO:21)) were used. For HVEM, primers (mHVBMIF, 5-ATGGAACCTCTCCCAGGATGGG-3 (SEQ ID NO:22); mHVEM1R, 5-TCAGTTGGAGGCTGTCTCCTCC-3 (SEQ ID NO:23)) were used. Each 3-step thermal cycle for routine PCR analysis included 30 s at 95 °C, 30 s at 60 °C, and 60 s at 72 °C; additional PCR reactions were performed for 35 cycles. PCR products were visualized by ethidium bromide staining of agarose gels and identified by size markers. A negative control containing all reagents except cDNA was included in each PCR analysis.

*In-situ Hybridization (ISH).* LIGHT riboprobes were synthesized from T3 (sense probe) and T7 (anti-sense probe) promoters, labeled with biotin-UTP (Roche) and used to localize LIGHT mRNA expression within cardiac tissue sections by ISH, as described (Gao *et al*., 2000, J Clin Invest 105:35-44).

*RNase Protection Assays.* RNA was evaluated by the Riboquant system (PharMingen), using mouse template sets mCK-1 and mCK-3b for cytokines; mCK5 for chemokines; and mCR5 and mCR6 for CC and CXC chemokine receptors, respectively. A riboprobe for mouse CXCR3 was prepared in house (Topham *et al*., 1999, J Clin Invest 104:1549-57). Methods for in vitro transcription, riboprobe purification and use, plus densitometric analysis and normalization of data to L32 and GAPDH gene expression, were as described (Gao *et al*., 2000, J Clin Invest 105:35-44).

*Immunopathology*. Hearts were fixed in formalin, paraffin-embedded, and stained with hematoxylin and eosin. Cryostat sections fixed in paraformaldehyde-lysine-periodate were stained by immunoperoxidase using mAbs to mouse leukocytes (Pharmingen), anti-LIGHT antibody (Santa Cruz Biotechnology) or isotype-matched controls (Hancock *et al*., 1996, Proc Natl Acad Sci USA 93:13967-72).

### 6.2 RESULTS AND DISCUSSION

Given the importance of LIGHT expression in the development of T cell activation *in vivo*, including in host T cell-dependent anti-tumor responses (Tamada *et al*., 2000, Nat. Med. 6:283-9; Zhai *et al*., 1998, J. Clin. Invest. 102:1142-51), we undertook a serial study of LIGHT and HVEM expression during the development of cardiac allograft rejection across a full MHC disparity. Total RNA was prepared from each recipient's heterotopic transplant and own native heart after collection on days 3 and 7 post-transplant, and HVEM and LIGHT mRNA expression were analyzed by RT-PCR. Negligible LIGHT or HVEM mRNA was detected in native hearts, but Northern analysis of LIGHT and HVEM expression in serial cardiac transplants rejected by day 7 indicated that expression of both LIGHT and HVEM were markedly upregulated at days 3 and 7 post-transplant in comparison with LIGHT and HVEM expression in the recipient's native heart. In situ hybridization analysis showed LIGHT mRNA expression in the graft LIGHT at day 7 post-transplant to be restricted to focal mononuclear cells with the morphology of leukocytes. HVEM mRNA was similarly localized to focal mononuclear cells; in addition, focal endothelial expression of HVEM was observed. Immunoperoxidase studies showed staining of infiltrating mononuclear leukocytes by anti-LIGHT IgG, including large cells with the morphologic features of inflammatory macrophages at day 7 post-transplant, including in cell clumps containing larger, branching DC-like cells.

To determine the role of LIGHT expression in allograft rejection, exon 1 of the LIGHT gene, which contains the ATG initiation codon, was disrupted by homologous recombination. A LIGHT gene-targeting construct was generated to replace exon 1 of LIGHT containing the initiating methionine (ATG) with the neomycin resistance gene (NEO). Southern analysis of LIGHT wild-type (+/+), heterozygous knockout (+/-) and homozygous knockout (-/-) mouse genomic DNA confirmed the replacement of exon 1 by the targeting construct. Northern analysis of LIGHT wild-type (+/+) and homozygous knockout (-/-) mouse liver RNA using a probe containing the entire coding region of LIGHT showed no detectable LIGHT mRNA expression in the LIGHT -/- liver tissue.

Mice heterologous and homozygous for the LIGHT mutation were normal in appearance, growth and fertility; had normal numbers of T and B cells, monocytes and granulocytes; and had normal lymphoid architecture by light microscopy.

Homozygous LIGHT-/- mice were used as recipients of fully MHC-disparate cardiac allografts. Whereas LIGHT+/+ mice rejected BALB/c cardiac allografts within 1 wk, LIGHT-/- mice maintained their grafts for an extra 3-4 days, which was about as effective as a sub-therapeutic dose of CsA (10 mg/kg/d) in mice (both p<0.05 vs. untreated LIGHT+/+ recipients). However, use of the same regimen of CsA in LIGHT-/- mice led to significantly prolonged engraftment (∼30 d, p<0.001), indicating a synergistic effect of CsA and LIGHT targeting on allograft survival.

Given concerns that gene-targeted mice might not always accurately reflect the full immune repertoire, the effect of inhibiting the HVEM-LIGHT pathway was also studied in wild-type allograft recipients. Accordingly, an HVEM-Ig fusion protein was constructed for therapeutic blockade of the effects of endogenous LIGHT on host HVEM+ T cells. Consistent with the effects of LIGHT targeting by homologous recombination in this strong MHC disparity, the studies in the wild-type allograft recipients demonstrated that HVEM-Ig alone or a combination of IgG and a subtherapeutic dose of CsA had negligible effects on allograft survival in LIGHT+/+ recipients (p>0.05). In contrast, the combination of HVEM-Ig plus low-dose CsA was markedly synergistic, significantly prolonging cardiac allograft survival (P < 0.001).

The levels of expression of multiple cytokines, chemokines and their receptors by host leukocytes vary during transplant rejection. RNase protection assays were used to examine the likely mechanisms by which targeting of LIGHT, especially in the context of concomitant CsA usage, induced prolonged allograft survival. The expression of intragraft Th1 and Th2-associated cytokines in control normal heart and allografts harvested at day 7 post-transplant was compared. Relative to expression in normal heart tissue, expression of IL-10, IFNγ, and IL-2 was upregulated in allograft recipients, including in LIGHT-deficient allograft recipients and allograft recipients treated with CsA at a dose of 10mg/kg/day. Treatment of LIGHT-/- mice with CsA suppressed the intragraft upregulation of multiple cytokine mRNAs, including IFN-γ, IL-2 and IL-10, as well as LT-β and TNF-α In contrast, expression of TGF-β2 was upregulated upon treatment of LIGHT-/- allograft recipients with subtherapeutic doses of CsA.

Consistent with these effects, expression of several IFN-γ-induced chemokines, including RANTES, MIP-1α, MIP-1β and IP-10, which are upregulated in cardiac tissue of allograft recipients receiving no therapy or single therapy relative to normal cardiac tissue, were markedly down-regulated in LIGHT-/- mice treated with CsA. Moreover, along with the decreased chemokine expression, LIGHT-/- mice treated with CsA had modest reductions in CC-chemokine receptor expression and markedly decreased expression of the chemokine receptor for IP-10, CXCR3, which is expressed by Th1 and Tc1 lymphocytes (Hancock *et al*., 2000, J. Exp. Med. 192:1515-20). Prior studies in this model showed that donor-derived IP-10 production (Hancock *et al*., 2001, J. Exp. Med. 193:975-80) and concomitant infiltration by CXCR3+ leukocytes (Hancock *et al*., 2000, J. Exp. Med. 192:1515-20) play central roles in the development of allograft rejection, such that targeting of either IP-10 production or CXCR3 expression markedly prolongs allograft survival. The IP-10/CXCR3 pathway is active during development of human cardiac allograft rejection (Melter *et al*., 2001, Circulation 104:2558-64). Thus, the combinatorial therapy methods described in the present study lead to a reduction of various molecular parameters that are indicative of allograft rejection.

The studies presented herein show marked synergism by combining immunosuppressive agents and blockade of HVEM-LIGHT costimulation. This is in contrast with other combination therapies, such as the use of CD154 mAb plus CsA (Smiley *et al*., 2000, Transplantation 70:415-9). As discussed above, the combination therapy methods of the present invention prevent modulate intragraft cytokine and chemokine production and decrease the infiltration of host immunocompetent cells, thereby inhibiting allograft rejection and prolonging survival of allograft recipients.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims.

Various publications are cited herein, the disclosures of which are incorporated by reference in their entireties.

## Claims

1. Use of
(i) an HVEM-LIGHT inhibitor; and
(ii) an immunosuppressive agent, in amounts effective for treating or preventing an immune disorder for preparing a pharmaceutical composition for treating or preventing an immune disorder in a patient.

2. The use of claim 1, wherein the immune disorder is allograft rejection, delayed-type hypersensitivity, graft versus host disease, a drug allergy, atrophic gastritis, thyroiditis, allergic encephalomyelitis, autoimmune hemolytic anemia, sympathetic ophthalmia, systemic lupus erythematosus, rheumatoid arthiritis, multiple sclerosis, asthma, inflammatory bowel disease, or myasthenia gravis.

3. The use of claim 2, wherein the immune disorder is allograft rejection.

4. The use of claim 3, wherein the allograft is an organ transplant.

5. The use of claim 4, wherein the organ is a heart, kidney, liver, lung or pancreas.

6. The use of claim 3, wherein the allograft is a tissue transplant.

7. The use of claim 6, wherein the tissue is heart valve, endothelial, cornea, eye lens or bone marrow tissue.

8. The use of claim 3, wherein the allograft is a skin graft.

9. The use of claim 1 or 3, wherein the immunosuppressive agent is cyclosporine.

10. The use of claim 1 or 3, wherein the immunosuppressive agent is FK506.

11. The use of claim 1 or 3, wherein the immunosuppressive agent is a steroid.

12. The use of claim 11, wherein the steroid is a corticosteroid.

13. The use of claim 1 or 3, wherein the immunosuppressive agent is rapamycin.

14. The use of claim 1 or 3, wherein the immunosuppressive agent is an antibody.

15. The use of claim 1 or 3, wherein the immunosuppressive agent is an antiproliferative agent.

16. The use of claim 15, wherein the antiproliferative agent is azathioprine or mycophenolate moefitil.

17. The use of claim 1 or 3, wherein the immunosuppressive agent is prednisone.

18. The use of claim 1 or 3, wherein the immunosuppressive agent is a purine analog.

19. The use of claim 18, wherein the purine analog is methotrexate or mercaptopurine.

20. The use of claim1 or 3, wherein the HVEM-LIGHT inhibitor and the immunosuppressive agent are both administered concurrently.

21. The use of claim 20, wherein the HVEM-LIGHT inhibitor and the immunosuppressive agent are both administered daily.

22. The use of claim 1 or 3, wherein the HVEM-LIGHT inhibitor and the immunosuppressive agent are administered successively.

23. The use of claim 22, wherein the HVEM-LIGHT inhibitor is administered prior to administration of the immunosuppressive agent.

24. The use of claim 22, wherein the HVEM-LIGHT inhibitor is adminidstered following administration of the immunosuppressive agent.

25. The use of claim 22, wherein the HVEM-LIGHT inhibitor and the immunsuppressive agent are administered at a 1-day interval.

26. The use of claim 22, wherein the HVEM-LIGHT inhibitor and the immunosuppressive agent are administered at a 2-day interval.

27. The use of claim 22, wherein the HVEM-LIGHT inhibitor and the immunosuppressive agent are administered at a 3-day interval.

28. The use of claim 1 or 3, wherein the HVEM-LIGHT inhibitor is a soluble HVEM polypeptide.

29. The use of claim 28, wherein the soluble HVEM polypeptide is a fusion protein further comprising an Ig domain.

30. The use of claim 1 or 3, wherein the HVEM-LIGHT inhibitor is a soluble LIGHT polypeptide.

31. The use of claim 30, wherein the soluble LIGHT polypeptide is a fusion protein further comprising an Ig domain.

32. The use of claim 1 or 3, wherein HVEM-LIGHT inhibitor is not a herpesvirus gD protein, lymphotoxin-α protein, a lymphotoxin-β receptor protein, or a TR6 receptor protein.

33. The use of claim 1 or 3, wherein the HVEM-LIGHT inhibitor is an anti-HVEM antibody.

34. The use of claim 1 or 3, wherein the HVEM-LIGHT inhibitor is an anti-LIGHT antibody.

35. The use of claim 1 or 3, wherein HVEM-LIGHT inhibitor is administered in form of gene therapy.

36. The use of claim 1 or 3, wherein the amounts of the HVEM-LIGHT inhibitor and immunosuppressive agent are synergistic.

37. The use of claim 36, wherein the amount of HVEM-LIGHT inhibitor or immunosuppressive agent, if administered alone, is not effective for treating allograft rejection.

38. The use of claim 36, wherein the amount of each of the HVEM-LIGHT inhibitor and immunosuppressive agent, if administered alone, is not effective for treating allograft rejection.

39. The use of claim 1 or 3, wherein the HVEM-LIGHT inhibitor is conjugated to the immunosuppressive agent.

40. A pharmaceutical composition comprising:
a. an HVEM-LIGHT inhibitor;
b. an immunosuppressive agent; and
c. a pharmaceutically acceptable carrier;
wherein the HVEM-LIGHT inhibitor and the immunosuppressive agent are present in amounts effective to prevent or treat allograft rejection.

41. A kit comprising in one or more containers:
a. HVEM-LIGHT inhibitor:
b. an immunosuppressive agent; and
c. instructions for the use of the HVEM-LIGHT inhibitor and the immunosuppressive agent in the prevention or treatment of allograft rejection.

42. An isolated nucleic acid molecule selected from the group consisting of:
a. a nucleic acid molecule having a nucleotide sequence which is at least 90% identical to the nucleotide sequence of SEQ ID NO:3 or a complement thereof as determined using the BLAST algorithm;
b. a nucleic acid molecule comprising at least 15 nucleotide residues and having a nucleotide sequence identical to at least 15 consecutive nucleotide residues of residues 1-424 of SEQ ID NO:3 or a complement thereof;
c: a nucleic acid molecule which encodes a polypeptide comprising the amino acid sequence of SEQ ID NO:4;
d. a nucleic acid molecule which encodes a fragment of a polypeptide comprising the amino acid sequence of any of SEQ ID NO:4, wherein the fragment comprises at least 12 consecutive amino acid residues of residues 1-80 of SEQ ID NO:4; and
e. a nucleic acid molecule which encodes a naturally occurring allelic variant of a polypeptide comprising the amino acid sequence of SEQ ID NO:4, wherein the nucleic acid molecule hybridizes under stringent conditions with a nucleic acid molecule consisting of the nucleotide sequence of any of residues 1-424 of SEQ ID NO:3 or a complement thereof.

43. The isolated nucleic acid molecule of claim 42, which is selected from the group consisting of:
a. a nucleic acid having the nucleotide sequence of SEQ ID NO:3 or a complement thereof; and
b. a nucleic acid molecule which encodes a polypeptide having the amino acid sequence of any of SEQ ID NO:4 or a complement thereof.

44. The isolated nucleic acid molecule of claim 44, wherein the fragment comprises at least 15 consecutive amino acid residues of residues 1-80 of SEQ ID NO:4.

45. The isolated nucleic acid molecule of claim 44, wherein the fragment comprises at least 20 consecutive amino acid residues of residues 1-80 of SEQ ID NO:4.

46. The isolated nucleic acid molecule of claim 44, wherein the fragment comprises at least 30 consecutive amino acid residues of residues 1-80 of SEQ ID NO:4.

47. The isolated nucleic acid molecule of claim 44, wherein the fragment comprises at least 40 consecutive amino acid residues of residues 1-80 of SEQ ID NO:4.

48. The isolated nucleic acid molecule of claim 44, wherein the fragment comprises at least 50 consecutive amino acid residues of residues 1-80 of SEQ ID NO:4.

49. The isolated nucleic acid molecule of claim 42, 43, or 44, further comprising vector nucleic acid sequences.

50. The isolated nucleic acid molecule of claim 42, 43, or 44, further comprising nucleic acid sequences encoding a heterologous polypeptide.

51. A host cell which contains the nucleic acid molecule of claim 42, 43, or 44.

52. The host cell of claim 51 which is a mammalian host cell.

53. The host cell of claim 51 which is a non-mammalian host cell.

54. An isolated polypeptide selected from the group consisting of:
a. a fragment of a polypeptide comprising the amino acid sequence of SEQ ID NO:4; wherein the fragment comprises at least 12 consecutive amino acid residues of residues 1-80 of SEQ ID NO:4;
b. a naturally occurring allelic variant of a polypeptide comprising the amino acid sequence of any of SEQ ID NO:4, wherein the polypeptide is encoded by a nucleic acid molecule which hybridizes with a nucleic acid molecule consisting of the nucleotide sequence of residues 1-424 of SEQ ID NO:3 or a complement thereof under stringent conditions; and
c. a polypeptide which is encoded by a nucleic acid molecule comprising a nucleotide sequence which is at least 90% identical to a nucleic acid consisting of residues 1-424 of SEQ ID NO:3 or a complement thereof as determined using the BLAST algorithm.

55. The isolated polypeptide of claim 54 having the amino acid sequence of SEQ ID NO:4.

56. The isolated polypeptide of claim 54, wherein the fragment comprises at least 15 consecutive amino acid residues of residues 1-80 of SEQ ID NO:4.

57. The isolated polypeptide of claim 56, wherein the fragment comprises at least 20 consecutive amino acid residues of residues 1-80 of SEQ ID NO:4.

58. The isolated polypeptide of claim 56, wherein the fragment comprises at least 30 consecutive amino acid residues of residues 1-80 of SEQ ID NO:4.

59. The isolated polypeptide of claim 56, wherein the fragment comprises at least 40 consecutive amino acid residues of residues 1-80 of SEQ ID NO:4.

60. The isolated polypeptide of claim 56, wherein the fragment comprises at least 50 consecutive amino acid residues of residues 1-80 of SEQ ID NO:4.

61. The isolated polypeptide of claim 54, wherein the amino acid sequence of the polypeptide further comprises heterologous amino acid residues.

62. An antibody which selectively binds with the polypeptide of claim 54, which antibody does not bind to a polypeptide comprising SEQ ID NO:2.

63. A method for producing a polypeptide selected from the group consisting of:
a. a fragment of a polypeptide comprising the amino acid sequence of SEQ ID NO:4; wherein the fragment comprises at least 12 consecutive amino acid residues of residues 1-80 of SEQ ID NO:4;
b. a naturally occurring allelic variant of a polypeptide comprising the amino acid sequence of any of SEQ ID NO:4, wherein the polypeptide is encoded by a nucleic acid molecule which hybridizes with a nucleic acid molecule consisting of the nucleotide sequence of residues 1-424 of SEQ ID NO:3 or a complement thereof under stringent conditions; and
c. a polypeptide which is encoded by a nucleic acid molecule comprising a nucleotide sequence which is at least 90% identical to a nucleic acid consisting of residues 1-424 of SEQ ID NO:3 or a complement thereof as determined using the BLAST algorithm.
said method comprising culturing the host cell of claim 51 under conditions in which the nucleic acid molecule is expressed.
